# EUROPEAN PATENT APPLICATION

(11) **EP 4 219 757 A1**
(43) Date of publication of application: **02.08.2023**
(21) Application number: 20954964.1
(22) Date of filing: 13.11.2020
(51) Int. Cl.: C12Q 1/686, C12N 15/11

(54) **MULTIPLEX NUCLEIC ACID DETECTION METHOD, AND COMBINATION AND KIT**

(30) Priority: 23.09.2020 CN 202011008766
(71) Applicant: Maccura Biotechnology Co., Ltd., Chengdu Sichuan 611731 (CN)
(72) Inventor: ZHAO, Yuhang, Chengdu, Sichuan 611731 (CN); ZHAN, Haomiao, Chengdu, Sichuan 611731 (CN); HUANG, Mei, Chengdu, Sichuan 611731 (CN)
(74) Representative: Dr. Gassner & Partner mbB
(86) International application number: PCT/CN2020/128663
(87) International publication number: WO 2022/062120

(57) **Abstract**

Disclosed in the present invention are a multiplex nucleic acid detection method, a composition and a kit. The method comprises a multiplex nucleic acid detection method, which comprises the following steps: mixing a composition containing a first primer set and a first probe with a sample from a subject; amplifying a target sequence that may be present in the sample; performing a melting curve analysis to an amplified product in a corresponding detection channel; and judging whether one or more targets are present, according to a result of the melting curve analysis.

## Description

### Field of the Invention

The invention relates to the field of molecular biology, in particular to a solution for detection of multiplex target melting curves.

### Background of the Invention

Polymerase chain reaction (PCR) is a molecular biological technology that performs DNA replication via enzyme without using living organisms. PCR is usually used in medical and biological research laboratories to undertake a variety of tasks, such as diagnosis of infectious diseases, gene cloning, phenotypic identification of laboratory animals, research on transcriptomes, detection of genetic diseases, identification of gene fingerprints, paternity test, etc. Since PCR has an incomparable replication ability and accuracy, it is considered by the molecular biologists as a preferred method for nucleic acid detection. In the late 1990s, a real-time fluorescence quantitative PCR (qPCR) technology and related products launched by ABI in the United States further developed PCR to be a highly sensitive, highly specific and accurately quantitative nucleic acid sequence analysis technology.

At present, a primer-probe design method that is widely used in qPCR platform is mainly a TagMan hydrolysis probe method, a working principle of which is mainly characterized by using an oligonucleotide probe that can specifically bind with a template and is labelled with a fluorescence group (donor) and a quencher group (receptor) at both ends, and by designing a specific PCR primer upstream and downstream of the probe respectively. Before the start of PCR reaction, due to a principle of fluorescence resonance energy transfer (FRET), a fluorescence signal emitted by the fluorescence group at one end of the TagMan probe is absorbed by the quencher group at the other end, such that the fluorescence signal cannot be detected by an instrument; but after the start of PCR amplification, the TagMan probe specifically binds with the template, and when a DNA polymerase (Tagase) extends to the site of the template to which the probe binds, the TagMan probe will be cleaved due to the 5-3' exonuclease activity of the DNA polymerase (Tagase), such that the fluorescence group labelled on the probe will be far away from the quencher group and a FRET structure cannot be formed any more, and thus a signal emitted by the fluorescence group can be detected by the instrument.

With the increasing application of PCR technology and the increasing demand for high-throughput and rapid detection, it is usually expected to achieve multiplex target PCR detection in one reaction system. In general, multiplex detection is formed by setting multiple TaqMan hydrolysis probes labelled with fluorescent groups having different wavelengths. However, depending on the number of fluorescent channels of a detection instrument, only about quadruple different targets may be detected at most by means of this method.

In order to realize super-multiplex PCR detection, in the prior art, the PCR detection technology is usually combined with other methods. It is reported that the PCR detection technology is combined with a hybridization chip to perform multiplex target detection via different amplified product lengths. For example, Chinese patent CN107090519A discloses a detection method of multiplex RT-PCR combined with a gene chip detection kit for common respiratory pathogens, comprsing: due to a principle of nucleic acid molecular hybridization, arranging and fixing single-stranded probes specific to individual targets on the gene chip in a specific order to form a probe array, and then allowing multiple PCR products to be detected to be hybridized with the probe array, such that the target amplified products are hybridized with the probes on the chip to emit fluorescence signals. Although 20 kinds of respiratory pathogens can be detected at the same time by means of this method, the gene chip is prepared by a complex process and is expensive, which is not beneficial to clinical expansion and development; and the process for detection is cumbersome, during which opening and cleaning steps are required to be repeated for many times, such that it is easy to cause pollution and lead to false positive results.

It is also reported that PCR detection technology is combined with capillary electrophoresis to perform multiplex target detection via different amplified product lengths. For example, Chinese patent CN103074450A discloses a kit for simultaneous detection of 30 kinds of diarrhoeal pathogenic bacteria and detection method thereof. The method combines PCR amplification and capillary electrophoresis, and comprises taking out the PCR amplified products and performing capillary electrophoresis analysis, and comparing the obtained electrophoregram with a standard electrophoregram to determine the types of diarrhoeal pathogenic bacteria. However, during this method, a capillary electrophoresis instrument is also required for the analysis of products in addition to a PCR amplification detection instrument, such that the detection cost is greatly increased, which is not beneficial to clinical expansion and application.

Therefore, there is a clinically urgent need for a method capable of performing efficient and low-cost multiplex molecular detection, so as to quickly and effectively detect a variety of clinically common targets such as bacteria, viruses, gene mutations, etc.

### Summary of the Invention

In order to solve the above problems, the inventors have made a research on the design of primer and probe for qPCR detection technology and have proposed a new solution for multiplex target detection, thereby achieving the present invention.

Accordingly, in a first aspect, the present invention provides a multiplex target detection method, comprising the following steps:
mixing a composition containing a first primer set and a first probe with a sample from a subject;
amplifying a target sequence that may be present in the sample;
performing a melting curve analysis to an amplified product in a corresponding detection channel;
and judging whether one or more targets are present, according to a result of the melting curve analysis,
wherein, the first primer set comprises an upstream primer and a downstream primer both of which are specific to a first target, and an upstream primer and a downstream primer both of which are specific to a second target,
wherein, the upstream primer specific to the first target comprises a target sequence binding region located at a 3' end and a first signal detection region located upstream of the target sequence binding region; and the downstream primer specific to the first target comprises a target sequence binding region located at a 3' end,
wherein, the upstream primer specific to the second target comprises a target sequence binding region located at a 3' end and a second signal detection region located upstream of the target sequence binding region; and the downstream primer specific to the second target comprises a target sequence binding region located at a 3' end,
wherein, the first signal detection region and the second signal detection region are designed to be not complementarily paired with a target sequence,
and wherein, all or part of the sequence of the first probe is the same as that of the first signal detection region, and all or part of the sequence of the first probe is the same as that of the second signal detection region, such that amplified products can be amplified (annealed and extended) after performing amplification using the first primer set; and the first probe comprises a first detection group and a second detection group, and the first detection group and the second detection group can generate signal change after being incorporated into double-stranded structures.

It can be understood that, in a process of PCR amplification, individual primer pairs specific to different targets specifically bind with their respective target sequences and are extended to produce pre-amplified products, and then the probe may be complementarily paired with the pre-amplified products for continuous PCR amplification. The first detection group and the second detection group are incorporated into the generated double-stranded amplified products, to generate signal change which can be detected by an instrument. After the PCR amplification is completed, the melting curve analysis is performed. In a process of temperature change, the double strands of the PCR products comprising the first detection group and the second detection group will be molten at a certain temperature, thereby producing signal change which can be detected by an instrument. By combining the types of detection groups contained in the probe and the different melting temperatures of the PCR products of individual targets, it is possible to achieve multiplex target detection.

In a variant of the first aspect of the present invention, the upstream primer specific to the first target comprises a target sequence binding region located at a 3' end; and the downstream primer specific to the first target comprises a target sequence binding region located at a 3' end and a first signal detection region located upstream of the target sequence binding region,
and wherein, the upstream primer specific to the second target comprises a target sequence binding region located at a 3' end; and the downstream primer specific to the second target comprises a target sequence binding region located at a 3' end and a second signal detection region located upstream of the target sequence binding region

In another variant of the first aspect of the present invention, the upstream primer specific to the first target comprises a target sequence binding region located at a 3' end and a first signal detection region located upstream of the target sequence binding region; and the downstream primer specific to the first target comprises a target sequence binding region located at a 3' end,
and wherein, the upstream primer specific to the second target comprises a target sequence binding region located at a 3' end; and the downstream primer specific to the second target comprises a target sequence binding region located at a 3' end and a second signal detection region located upstream of the target sequence binding region.

In some embodiments, the method of the present invention further comprises a step of quantifying the detected target. For example, the target may be quantified by detecting real-time fluorescence signals.

In a preferred embodiment, the composition of the present invention further comprises a second primer set and a second probe,
wherein, the second primer set comprises an upstream primer and a downstream primer both of which are specific to a third target, and an upstream primer and a downstream primer both of which are specific to a fourth target,
wherein, the upstream primer specific to the third target or the downstream primer specific to the third target comprises a third signal detection region located upstream of a target sequence binding region; and the upstream primer specific to the fourth target or the downstream primer specific to the fourth target comprises a fourth signal detection region located upstream of a target sequence binding region,
wherein, the third signal detection region and the fourth signal detection region are designed to be not complementarily paired with a target sequence,
and wherein, all or part of the sequence of the second probe is the same as that of the third signal detection region, and all or part of the sequence of the second probe is the same as that of the fourth signal detection region, such that amplified products can be amplified (annealed and extended) after performing amplification using the second primer set; and the second probe comprises a third detection group and a fourth detection group, and the third detection group and the fourth detection group can generate signal change after being incorporated into double-stranded structures; and a type (e.g., wavelength) of a signal generated by the second probe is different from that of a signal generated by the first probe.

It can be understood that, by the above method, it is possible, in one detection channel, to detect signals corresponding to the first probe and perform a melting curve analysis, so as to judge whether targets corresponding to the first primer set are present; meanwhile, it is possible, in another detection channel, to detect signals corresponding to the second probe and perform a melting curve analysis, so as to judge whether targets corresponding to the second primer set are present.

Similarly, the composition of the present invention may further comprise a third probe and even a fourth probe, so as to detect, in their respective detection channels, the targets to which their corresponding third and fourth primer sets are specific.

In some embodiments, each primer set in the composition of the first aspect may contain a plurality of upstream and downstream primer pairs specific to three, four or more targets, and these upstream primers or the downstream primers each are provided with their own signal detection regions; and in each primer set, the corresponding probe can amplify (anneal and extend) the amplified product of this primer set, but cannot amplify (anneal and extend) the amplified products of other primer sets.

It can be understood that, in a process of a melting curve analysis, different targets corresponding to each primer set are distinguished in each detection channel according to different melting temperatures.

Adjusting the melting temperature of the amplified product is known by those skilled in the art. For example, the melting temperatures of amplified products of different targets may be distinguished by adjusting a length of spacer bases between the signal detection region and the target sequence binding region (for example, adding a certain number of G/C bases). Specifically, when a number of the spacer bases is increased, the melting temperature will be increased accordingly; and when the number of the spacer bases is decreased, the melting temperature will be decreased accordingly.

In the method of the first aspect, the melting curve analysis may be performed in a range of 60-95 °C. In each detection channel, a melting temperature difference between the amplified products corresponding to individual targets is at least 2 °C.

In addition, the first aspect of the present invention further provides a composition for multiplex target detection, comprising:
a first primer set, comprising an upstream primer and a downstream primer both of which are specific to a first target, and an upstream primer and a downstream primer both of which are specific to a second target; and
a first probe, all or part of the sequence thereof is the same as that of a first signal detection region, and all or part of the sequence of the first probe is the same as that of a second signal detection region, such that amplified products can be amplified (annealed and extended) after performing amplification to a target sequence using the first primer set; and the first probe comprises a first detection group and a second detection group, and the first detection group and the second detection group can generate signal change after being incorporated into double-stranded structures,
wherein, the first signal detection region is located upstream of a target sequence binding region of the upstream primer specific to the first target or the downstream primer specific to the first target, and the second signal detection region is located upstream of a target sequence binding region of the upstream primer specific to the second target or the downstream primer specific to the second target; and the first signal detection region and the second signal detection region are designed to be not complementarily paired with a target sequence.

In the present invention, the probe may have a length of 15-30 nt.

In the present invention, the upstream primer or downstream primer comprising the signal detection region may have a length of 30-80 nt.

In the present invention, the upstream primer or downstream primer not comprising the signal detection region may have a length of 15-30 nt.

In some embodiments, in the composition of the first aspect, the signal detection regions in each primer set are the same as each other. For example, in the first primer set, the first signal detection region is the same as the second signal detection region.

In some embodiments, within each primer set in the composition of the first aspect, the target sequence binding region may be spaced apart from the signal detection region located upstream of the target sequence by 0-40 nt.

In a specific embodiment, the first detection group and the second detection group on the probe of the first aspect of the present invention are not located at 3' ends.

In a specific embodiment, the first detection group and the second detection group on the probe in the first aspect of the present invention are spaced apart from each other by 5-25 nt.

In a second aspect, the present invention provides a method for multiplex target detection, including the following steps:
mixing a composition containing a first primer set and a first probe with a sample from a subject;
amplifying a target sequence that may be present in the sample;
performing a melting curve analysis to an amplified product in a corresponding detection channel;
and judging whether one or more targets are present, according to a result of the melting curve analysis,
wherein, the first primer set comprises an upstream primer and a downstream primer both of which are specific to a first target, and an upstream primer and a downstream primer both of which are specific to a second target,
wherein, the upstream primer specific to the first target comprises a target sequence binding region located at a 3' end and a first signal detection region located upstream of the target sequence binding region; and the downstream primer specific to the first target comprises a target sequence binding region located at a 3' end,
wherein, the upstream primer specific to the second target comprises a target sequence binding region located at a 3' end and a second signal detection region located upstream of the target sequence binding region; and the downstream primer specific to the second target comprises a target sequence binding region located at a 3' end,
wherein, the first signal detection region and the second signal detection region are designed to be not complementarily paired with a target sequence,
and wherein, all or part of the sequence of the first probe is the same as that of the first signal detection region, and all or part of the sequence of the first probe is the same as that of a second signal detection region, so as to be hybridized with amplified products after performing amplification using the first primer set; and the first probe comprises a first detection group and a second detection group, and the first detection group and the second detection group can generate signal change after hybridization, and the 3' end of the first probe cannot be extended as a primer.

It can be understood that, in a process of PCR amplification, individual primer pairs specific to different targets specifically bind with their respective target sequences and are extended to produce amplified products, and then the probe may be hybridized with the amplified products, such that a distance between the first detection group and the second detection group is changed, to generate signal change which can be detected by an instrument.

After the PCR amplification is completed, the melting curve analysis is performed. In a process of temperature change, the probe which hybridizes with the amplified products will be molten at a certain temperature and be separated from the product strands, such that a distance between the first detection group and the second detection group is changed, to generate signal change which can be detected by an instrument. By combining the type of the first detection group contained in the probe and the different melting temperatures of the probes to which are individual targets bind, it is possible to achieve multiplex target detection.

In a variant of the second aspect of the present invention, the upstream primer specific to the first target comprises a target sequence binding region located at a 3' end; and the downstream primer specific to the first target comprises a target sequence binding region located at a 3' end and a first signal detection region located upstream of the target sequence binding region,
and wherein, the upstream primer specific to the second target comprises a target sequence binding region located at a 3' end; and the downstream primer specific to the second target comprises a target sequence binding region located at a 3' end and a second signal detection region located upstream of the target sequence binding region

In another variant of the second aspect of the present invention, the upstream primer specific to the first target comprises a target sequence binding region located at a 3' end and a first signal detection region located upstream of the target sequence binding region; and the downstream primer specific to the first target comprises a target sequence binding region located at a 3' end,
and wherein, the upstream primer specific to the second target comprises a target sequence binding region located at a 3' end; and the downstream primer specific to the second target comprises a target sequence binding region located at a 3' end and a second signal detection region located upstream of the target sequence binding region.

In a preferred embodiment, a composition of the second aspect further comprises a second primer set and a second probe,
wherein, the second primer set comprises an upstream primer and a downstream primer both of which are specific to a third target, and an upstream primer and a downstream primer both of which are specific to a fourth target,
wherein, the upstream primer specific to the third target or the downstream primer specific to the third target comprises a third signal detection region located upstream of a target sequence binding region; and the upstream primer specific to the fourth target or the downstream primer specific to the fourth target comprises a fourth signal detection region located upstream of a target sequence binding region,
wherein, the third signal detection region and the fourth signal detection region are designed to be not complementarily paired with a target sequence,
and wherein, all or part of the sequence of the second probe is the same as that of the third signal detection region, and all or part of the sequence of the second probe is the same as that of the fourth signal detection region, so as to be capable of hybridizing with amplified products after performing amplification using the second primer set; the 3' end of the second probe cannot be extended as a primer; and the second probe comprises a third detection group and a fourth detection group, and the third detection group and the fourth detection group can generate signal change after hybridization, and a type (e.g., wavelength) of a signal generated by the second probe is different from that of a signal generated by the first probe.

It can be understood that, by the above method, it is possible, in one detection channel, to detect signals corresponding to the first probe and perform a melting curve analysis, so as to judge whether targets corresponding to the first primer set are present; meanwhile, it is possible, in another detection channel, to detect signals corresponding to the second probe and perform a melting curve analysis, so as to judge whether targets corresponding to the second primer set are present.

Similarly, the composition of the present invention may further comprise a third probe and even a fourth probe, so as to detect, in their respective detection channels, the targets to which their corresponding third and fourth primer sets are specific.

In some embodiments, each primer set in the composition of the second aspect may contain a plurality of upstream and downstream primer pairs specific to three, four or more targets, and these upstream primers or the downstream primers each are provided with their own signal detection regions; and in each primer set, the corresponding probe can be hybridized with the amplified products of this primer set, but cannot be hybridized with the amplified products of other primer sets.

It can be understood that, in a process of a melting curve analysis, different targets corresponding to each primer set are distinguished in each detection channel according to different melting temperatures.

Adjusting the melting temperature of the probe and the amplified product is known by those skilled in the art. For example, the melting temperatures of amplified products of different targets may be distinguished by adjusting a mismatching degree between the probe and the complementary sequence of the signal detection region. Specifically, when the probe in the second aspect of the present invention is completely matched with the complementary sequence of the signal detection region, the melting temperature is the highest; and when there are mismatched bases between the probe and the complementary sequence of the signal detection region, the melting temperature is decreased.

In the method of the second aspect, the melting curve analysis may be performed in a range of 45-85 °C. In each detection channel, a melting temperature difference between the amplified products corresponding to individual targets is at least 2 °C.

In addition, in the second aspect, the present invention further provides a composition for multiplex target detection, comprising:
a first primer set, comprising an upstream primer and a downstream primer both of which are specific to a first target, and an upstream primer and a downstream primer both of which are specific to a second target; and
a first probe, all or part of the sequence thereof is the same as that of a first signal detection region, and all or part of the sequence thereof is the same as that of a second signal detection region, so as to be capable of hybridizing with amplified products of the first primer set; and the first probe comprises a first detection group and a second detection group, and the first detection group and the second detection group can generate signal change after hybridization, and the 3' end of the first probe cannot be extended as a primer,
wherein, the first signal detection region is located upstream of a target sequence binding region of the upstream primer specific to the first target or the downstream primer specific to the first target, and the second signal detection region is located upstream of a target sequence binding region of the upstream primer specific to the second target or the downstream primer specific to the second target; and the first signal detection region and the second signal detection region are designed to be not complementarily paired with a target sequence.

In some embodiments, within each primer set in the composition of the first aspect, the target sequence binding region and the signal detection region located upstream thereof may be spaced apart from each other by 0-10 nt.

In some embodiments, the composition of the present invention may further comprise an amplification reagent for PCR reaction.

In some embodiments, the first detection group or the second detection group on the probe of the second aspect of the present invention is located at a 3' end.

In some embodiments, the 3' end of the probe according to the second aspect of the present invention is blocked. It may be blocked by using a method well known in the art, for example, a C3, C6, C9 or C12 spacer modification may be performed at the 3' end; and for another example, a ddC modification may be performed at the 3' end.

In a specific embodiment, the first detection group and the second detection group on the probe according to the second aspect of the present invention may be spaced apart from each other by 5-25nt.

The method and composition of the present invention have the following beneficial effects:
the present invention allows simultaneous two-dimensional analysis of detection channel and melting temperature in a single-tube reaction, so as to detect the number of targets obtained by multiplying a number of detection channels by a number of melting temperature characteristics;
in the present invention, only one probe is used in each detection channel, which greatly reduces fluorescence background in the PCR reaction and improves reaction sensitivity;
in the design method of the present invention, only the primer and the target sequence are complementarily paired, such that when there are many high variation regions in the target sequence, such as virus or bacterial genomes, the present invention has a better tolerance and a lower design difficulty;
in the present invention, the detection is completed by only using a nucleic acid amplification analyzer, without the aid of additional downstream instruments, and without the need to divide reactions through chips or multiplex reaction tubes, which greatly reduces the cost of instruments and consumables and the complexity of operations, while reduces the number of probes in the reagent, greatly reduces the cost of reagents, and provides a possibility for large-scale expansion;
the present invention can achieve multiplex detection in a single reaction tube with less sample consumption, such that it is particularly suitable for the detection of rare samples, and can greatly increase the concentration of samples added for detection reaction and improve the detection sensitivity; in addition, since the single-tube reaction can be achieved, a possibility for subsequent molecular POCT-based detection is provided;
in the method of the present invention, a completely closing is achieved after the completion of sample addition, without the need to open the cover for subsequent detection, thereby greatly reducing a possibility of pollution to the experimental environment; and
the present invention has a very low requirement for the length of the target sequence, so as to be capable of improving the sensitivity for the detection of short fragment nucleic acids.

### Brief Description of the Drawings

Figure 1 shows a melting curve representing ADV positive in a FAM channel according to the multiplex target detection of Example 2;
Figure 2 shows a melting curve representing IBV positive in a FAM channel according to the multiplex target detection of Example 2;
Figure 3 shows a melting curve representing MP positive in a ROX channel according to the multiplex target detection of Example 2;
Figure 4 shows a melting curve representing SARS-CoV-2 positive in a ROX channel according to the multiplex target detection of Example 2;
Figure 5 shows a melting curve representing RSV positive in a Cy5 channel according to the multiplex target detection of Example 2;
Figure 6 shows a melting curve representing IAV positive in a Cy5 channel according to the multiplex target detection of Example 2;
Figure 7 shows double melting peaks representing simultaneous appearance of ADV and IBV in a reaction hole with addition of ADV and IBV positive templates in a FAM channel in Example 3;
Figure 8 shows double melting peaks representing simultaneous appearance of MP and SARS-CoV-2 in a reaction hole with addition of MP and SARS-CoV-2 positive templates in a ROX channel in Example 3;
Figure 9 shows double melting peaks representing simultaneous appearance of RSV and IAV in a reaction hole with addition of RSV and IAV positive templates in a Cy5 channel in Example 3;
Figure 10 shows a melting curve representing IBV positive in a FAM channel according to the multiplex target detection of Example 5;
Figure 11 shows a melting curve representing IAV positive in a FAM channel according to the multiplex target detection of Example 5;
Figure 12 shows a melting curve representing ADV positive in ROX channel according to the multiplex target detection of Example 5;
Figure 13 shows a melting curve representing RSV positive in a ROX channel according to the multiplex target detection of Example 5;
Figure 14 shows a melting curve representing MP positive in a Cy5 channel according to the multiplex target detection of Example 5;
Figure 15 shows a melting curve representing SARS-CoV-2 positive in a Cy5 channel according to the multiplex target detection of Example 5;
Figure 16 shows double melting peaks representing simultaneous appearance of IAV and IBV in a reaction hole with addition of IAV and IBV positive templates in a FAM channel in Example 6;
Figure 17 shows double melting peaks representing simultaneous appearance of ADV and RSV in a reaction hole with addition of ADV and RSV positive templates in a ROX channel in Example 6;
Figure 18 shows double melting peaks representing simultaneous appearance of MP and SARS-CoV-2 in a reaction hole with addition of MP and SARS-CoV-2 positive templates in a Cy5 channel in Example 6.
Figure 19 shows a melting curve representing IBV positive in a FAM channel according to the multiplex target detection of Example 7;
Figure 20 shows a melting curve representing IAV positive in a FAM channel according to the multiplex target detection of Example 7;
Figure 21 shows a melting curve representing ADV positive in a ROX channel according to the multiplex target detection of Example 7;
Figure 22 shows a melting curve representing RSV positive in a ROX channel according to the multiplex target detection of Example 7;
Figure 23 shows a melting curve representing MP positive in a Cy5 channel according to the multiplex target detection of Example 7;
Figure 24 shows a melting curve representing SARS-CoV-2 positive in a Cy5 channel according to the multiplex target detection of Example 7;
Figure 25 shows double melting peaks representing simultaneous appearance of IAV and IBV in a reaction hole with addition of IAV and IBV positive templates in aFAM channel in Example 8;
Figure 26 shows double melting peaks representing simultaneous appearance of ADV and RSV in a reaction hole with addition of ADV and RSV positive templates in a ROX channel in Example 8;
Figure 27 shows double melting peaks representing simultaneous appearance of MP and SARS-CoV-2 in a reaction hole with addition of MP and SARS-CoV-2 positive templates in a Cy5 channel in Example 8.
Figure 28 shows a melting curve representing IBV positive in a FAM channel according to the multiplex target detection of Example 9;
Figure 29 shows a melting curve representing IAV positive in a FAM channel according to the multiplex target detection of Example 9;
Figure 30 shows a melting curve representing ADV positive in a ROX channel according to the multiplex target detection of Example 9;
Figure 31 shows a melting curve representing RSV positive in a ROX channel according to the multiplex target detection of Example 9;
Figure 32 shows a melting curve representing RhV positive in a Cy5 channel according to the multiplex target detection of Example 9;
Figure 33 shows a melting curve representing MP positive in a Cy5 channel according to the multiplex target detection of Example 9;
Figure 34 shows a melting curve representing SARS-CoV-2 positive in a Cy5 channel according to the multiplex target detection of Example 9;
Figure 35 shows three melting peaks representing simultaneous appearance of RhV, MP and SARS-CoV-2 in a reaction hole with addition of RhV, MP and SARS-CoV-2 positive templates in a Cy5 channel in Example 9; and
Figure 36 shows an amplification curve result (upper side) and a melting curve result (lower side) representing GAPDH positive in a VIC channel according to the multiplex target detection of Example 9.

### Detailed Description of the Embodiments

Hereafter, the technical solutions of the embodiments of the present invention will be clearly and completely described in combination with the accompanying drawings. Obviously, the described embodiments only represent some embodiments of the present invention, but are not exhaustive. Based on the described embodiments of the present invention, all the other embodiments that may be obtained by one of ordinary skill in the art without creative efforts will fall into the scope of protection of the present invention.

Throughout the description, unless otherwise specified, terms used herein should be construed that they have the same meanings as those commonly used in the art. Therefore, unless otherwise defined, all the technical and scientific terms have the same meanings as those generally understood by those skilled in the art to which the present invention pertains. In case of any contradiction, this description is intended to take precedence.

The present invention provides a multiplex nucleic acid detection method, comprising the following steps:
mixing a composition containing a first primer set and a first probe with a sample from a subject;
amplifying a target sequence that may be present in the sample;
performing a melting curve analysis to an amplified product in a corresponding detection channel;
and judging whether one or more targets are present, according to a result of the melting curve analysis,
wherein, the first primer set comprises an upstream primer and a downstream primer both of which are specific to a first target, and an upstream primer and a downstream primer both of which are specific to a second target,
wherein, the upstream primer specific to the first target or the downstream primer specific to the first target comprises a first signal detection region located upstream of a target sequence binding region,
wherein, the upstream primer specific to the second target or the downstream primer specific to the second target comprises a second signal detection region located upstream of a target sequence binding region,
wherein, the first signal detection region and the second signal detection region are designed to be not complementarily paired with a target sequence,
and wherein, all or part of the sequence of the first probe is the same as those of the first signal detection region and the second signal detection region, so as to be capable of forming double-stranded structures with complementary sequences of the first signal detection region and the second signal detection region respectively; and the first probe comprises a first detection group and a second detection group, and the first detection group and the second detection group can generate signal change after being incorporated into the double-stranded structures.

It can be understood that, for each target to be detected, the method of the present invention provides a corresponding primer pair. Each primer pair (the upstream primer or the downstream primer) is provided thereon with a signal detection region. The signal detection region is not complementarily paired with the target sequence, but has a sequence that is the same as all or part of the sequence of the probe of the present invention.

Herein, the term "nucleic acid" refers to a single-stranded and/or double-stranded polymer of nucleotide monomer(s), including but not limited to 2'- deoxyribonucleotides (DNA) and ribonucleotides (RNA) connected by an internucleotide phosphate diester bond or an internucleotide analogue. The nucleotide monomer in the nucleic acid may be called "nucleotide residue". The nucleic acid may be completely composed of deoxyribonucleotides, completely composed of ribonucleotides, or composed of chimeric mixtures thereof, and may include nucleotide analogues. The nucleotide monomer unit may include any one of the nucleotides described herein, including but not limited to nucleotides, and/or nucleotide analogues (such as, modified nucleotides). The size of the nucleic acid usually varies from a few nucleotide residues to thousands of nucleotide residues. Among others, "oligonucleotide" usually refers to a nucleotide polymer with a relatively short length (such as, less than 80). Unless otherwise indicated, when a nucleic acid sequence is presented, it should be understood that nucleotides are in an order of 5' to 3' from left to right. Unless otherwise indicated, "A" represents an adenine, "C" represents a cytosine, "G" represents a guanine, "T" represents a thymine, and "U" represents a uracil.

According to idiomatic expressions in the art, the length of the nucleic acid may be expressed by base pairs (abbreviated as "bp"), nucleotides (abbreviated as "nt") or kilobases (abbreviated as "kb").

Herein, the term "base complementary pairing" refers to a phenomenon that A and T, A and U, and G and C each are connected by hydrogen bonds to form a corresponding relationship therebetween. Accordingly, the term "base mismatching" refers to all the other pairing situations except those specified in the "base complementary pairing", such as mismatching of A and C, A and G, T and G, T and C, etc.

Herein, the term "primer" refers to an oligonucleotide that can "initiate" DNA synthesis through a template-dependent DNA polymerase, that is, for example, a 3' end of the oligonucleotide provides a free 3'-OH group, and more "nucleotides" may be connected to the 3'-OH group through the template-dependent DNA polymerase to establish a 3' to 5' phosphate diester bond, thereby using a deoxynucleotide triphosphate, and releasing a pyrophosphate.

Herein, the terms "target sequence", "target nucleic acid", "target nucleic acid sequence" or "target" may be used interchangeably and each refers to a part of nucleic acid sequence to be amplified, detected or amplified and detected, which may be annealed with a primer under annealing or amplification conditions.

The term "hybridization" refers to a base pairing interaction between two nucleic acids, which results in the formation of a double-stranded body. It should be understood that, in order to achieve hybridization, the two nucleic acids are not required to have 100% complementarity in the full length.

Herein, the term "upstream primer", also known as forward primer, is an oligonucleotide that is continuously extended along a negative strand. The term "downstream primer" used in the present invention, also known as reverse primer, is an oligonucleotide that is continuously extended along a positive strand. Among them, the positive strand (namely a sense strand, a positive-sense chain), also known as a coding chain, is generally located at an upper end of a double-stranded DNA, has a direction of 5'-3' from left to right, and has a base sequence substantially the same as a gene mRNA; and a primer that binds with this strand is a reverse primer. The negative strand (namely a nonsense strand), also known as a non-coding strand, is complementary to the positive strand; and a primer that binds with this strand is a forward primer. It should be understood that, when the designations of the positive-sense strand and an antisense strand are interchanged, the corresponding designations of the forward and reverse primer may also be interchanged accordingly.

Herein, the terms "upstream", "located upstream of.../upstream of...", "provided with...upstream of...", etc., in the context of describing a nucleic acid sequence, each refers to a part that is closer to a 5' end than a reference region on the same nucleic acid sequence, for example, the part may be adjacent to the reference region, or it may be spaced apart from the reference region by one or more bases. Accordingly, the terms "downstream", "located downstream of.../downstream of...", "provided with... downstream of...", etc. used herein, in the context of describing the nucleic acid sequence, refer to a part that is closer to a 3' end than the reference region on the same nucleic acid sequence, for example, the part may be adjacent to the reference region, or it may be spaced apart from the reference region by one or more bases. It should be understood that, unless otherwise stated, when the nucleic acid described is a double-stranded nucleic acid, the expressions of "upstream" and "downstream" are usually based on the 5' and 3' ends of a positive-sense strand.

Herein, the term "probe" refers to a labelled oligonucleotide used for detecting whether a target is present.

Herein, the hybridization may be implemented under strict conditions. The "strict conditions" may be any one of low strict conditions, medium strict conditions and high strict conditions. The "low strict conditions", for example, are conditions with 5 × SSC, 5 × Denhardt solution, 0.5% SDS, and 50% formamide, at 32 ° C; the "medium strict conditions", for example, are conditions with 5 × SSC, 5×Denhardt solution, 0.5% SDS, and 50% formamide, at 42 °C; and the "high strict conditions", for example, are conditions with 5 × SSC, 5 × Denhardt solution, 0.5% SDS, and 50% formamide, at 50 °C. Under these conditions, it is expected that, the higher the temperature is, the higher the possibility of effectively obtaining polynucleotides (such as DNA) with high homology is. Although there are many factors that affect a strictness of hybridization, such as temperature, probe concentration, probe length, ionic strength, time, salt concentration, etc., those skilled in the art can obtain a similar strictness by properly selecting these factors.

In a specific embodiment, the probe of the present invention may be selected from the group consisting of: an oligonucleotide that can form a curly shape due to molecular flexibility in single-stranded state, an oligonucleotide that can form a hairpin structure in single-stranded state, an oligonucleotide that can form a stem-loop structure in single-stranded state, an oligonucleotide that can form a pseudoknot structure in single-stranded state, and an oligonucleotide that can form a triplex structure in single-stranded state.

Herein, "an oligonucleotide that can form a curly shape due to molecular flexibility in single-stranded state" and "flexible probe" may be used interchangeably. The oligonucleotide forms a curly shape due to molecular flexibility in single-stranded state, but after it is extended to form double-stranded state by hybridization or annealing, it is fixed to form a relatively rigid double-helix structure due to hydrogen-bond interaction, such that a distance between the detection groups labelled on the oligonucleotide/probe is changed, thereby generating a detectable signal. For example, such oligonucleotides/probes may be obtained by referring to the recitations of US 9845492.

The probe in the second aspect of the present invention may also be called as a hybridization probe.

In some embodiments, one of the first (third) detection group and the second (fourth) detection group of the present invention may be a fluorescence group, and the other may be a quencher group or other groups that can generate signal change through fluorescence resonance energy transfer with the fluorescence group.

In an exemplary embodiment, the group that generates signal change through fluorescence resonance energy transfer may be Cy3 or Cy5, because they can generate fluorescence signal change through FRET effect therebetween.

In the present invention, the first detection group, for example, may be selected from the group consisting of FAM, HEX, VIC, ROX, Cy3, Cy5 and Cy5.5.

In the present invention, the second detection group, for example, may be selected from the group consisting of TAMRA, BHQ1, BHQ2, BHQ3, DABCYL, QXL and DDQI.

It should be noted that the probe of the present invention may be annealed and extended or hybridized with the amplified product of the corresponding primer set, and the detection group on the probe is thus incorporated into the resulted double strand. Compared with a single-stranded form, the double-stranded form of the probe may cause change (increase) of the distance between the detection groups, which is not caused by hydrolysis or release of the detection group.

In some embodiments, the composition of the present invention may be a kit, which further comprises an amplification reagent.

In the present invention, the term "amplification reagent" refers to a reagent used for PCR, including but not limited to dNTP, DNA polymerase, and some reagents that promote PCR reaction, such as KCl, MgCl₂, Tris-HCl, dithiothreitol (DTT), etc.

In the present invention, the individual components in the composition and kit may be present in the form of separate sub-packaging or pre-mixing.

In the present invention, the purpose of "pre-denaturation" and "denaturation" is to destroy a hydrogen bond between paired complementary bases on a double-stranded DNA, thereby allowing the double-stranded DNA to be separated into two single strands. For example, the single strand may be formed by heating a mixture containing the double-stranded DNA, for example, heating the mixture to 90 °C, 92 °C, 95 °C or 98 °C to dissociate double-stranded DNA. After dissociation, it is cooled to the room temperature or below. For another example, the single strand may also be formed by changing the ionic strength of a solution (for example, adding acid, alkali, salt, etc.) to break the hydrogen bond of the double strand DNA, and may also be formed by using enzymes (for example, a helicase) to dissociate the double-stranded DNA into single-stranded DNA.

In the present invention, the upstream primer and the downstream primer each may have a Tm value of 50 °C-80 °C, and a GC content of 40% - 80%.

In the present invention, the probe may have a Tm value of 50 °C-80- °C, and a GC content of 40%-80%.

In the method of the present invention, the specific reaction conditions of the amplifying step may be as follows: performing, pre-denaturation at 90 °C-96 °C for 5-15 minutes; performing, denaturation at 90 °C-95 °C for 10-60 seconds, and annealing and extension at 45 °C-75 °C for 30-90 seconds, for a total of 3-10 cycles; and performing, denaturation at 90 °C-95 °C for 10-60 seconds, and annealing and extension at 45 °C-75 °C for 30-90 seconds, for a total of 35-50 cycles. For another example, the specific reaction conditions of the amplifying step may be as follows: performing pre-denaturation at 90 °C-96 °C for 5-15 minutes; and performing, denaturation at 90 °C-95 °C for 10-60 seconds, and annealing and extension at 45 °C-75 °C for 30-90 seconds, for a total of 35-50 cycles.

In a specific embodiment, the specific reaction conditions of the amplifying step may be as follows: performing pre-denaturation at 95 °C for 3 minutes; performing, denaturation at 94 °C for 10 seconds, and annealing and extension at 61 °C for 15 seconds, for a total of 10 cycles; and performing, denaturation at 94 °C for 10 seconds, and annealing and extension at 55 °C for 15 seconds, for a total of 40 cycles.

In a specific embodiment, the specific reaction conditions for amplifying may be as follows: performing pre-denaturation at 95 °C for 3 minutes; performing, denaturation at 94 °C for 10 seconds, and annealing and extension at 60 °C for 15 seconds, for a total of 10 cycles; and performing, denaturation at 94 °C for 10 seconds, and annealing and extension at 56 °C for 15 seconds, for a total of 40 cycles.

In the first aspect of the present invention, the reaction conditions for the melting curve analysis after amplification may be as follows: performing, denaturation at 90 °C-96 °C for 10-60 seconds, annealing at 45 °C-55 °C for 60-120 seconds, and melting curve analysis at 50 °C-95 °C, with a temperature rise rate of 0.02-0.1 °C/s. Specifically, the reaction conditions for the melting curve analysis may be as follows: performing, denaturation at 94 °C for 10 seconds, annealing at 50 °C for 90 seconds, and melting curve analysis at 65 °C-95 °C, with a temperature rise rate of 0.05 °C/s.
In the second aspect of the present invention, the reaction conditions for the melting curve analysis after amplification may be as follows: performing, denaturation at 90 °C-95 °C for 10-60 seconds, annealing at 45 °C-55 °C for 60-120 seconds, and melting curve analysis at 45-85 °C, with a temperature rise rate of 0.02-0.1 °C/s. Specifically, the reaction conditions for the melting curve analysis may be as follows: perfoming, denaturation at 94 °C for 10 seconds, annealing at 50 °C for 120 seconds, and melting curve analysis at 50-90 °C, with a temperature rise rate of 0.03 °C/s.

In the first aspect of the present invention, in the case of containing signal detection region, the concentration of the upstream primer or the downstream primer in a reaction system may be, for example, 30 nM-150 nM, preferably 30 nM-120 nM, more preferably 40 nM. Otherwise, the concentration of upstream primer or downstream primer in a reaction system may be, for example, 150 nM-1200 nM, preferably 150 nM-450 nM, more preferably 240 nM.

In the first aspect of the present invention, the concentration of the probe in the reaction system may be, for example, 150 nM-1200 nM, preferably 150 nM-450 nM, more preferably 240 nM.

In the second aspect of the present invention, in the case of containing signal detection region, the concentration of the upstream primer or the downstream primer in a reaction system may be, for example,15 nM-150 nM, preferably 30 nM-90 nM, more preferably 45 nM. Otherwise, the concentration of upstream primer or downstream primer in a reaction system may be, for example, 150 nM-1500 nM, preferably 300 nM-600 nM, more preferably 250 nM.

In the second aspect of the present invention, the concentration of the probe in the reaction system may be, for example, 150 nM-1800 nM, preferably 300 nM-900 nM, more preferably 250 nM.

In the present invention, the expressions such as "first", "second", "third" and "fourth" are only used for the purpose of describing, so as to distinguish the defined substances, and are not intended to limit the order or priority in any way.

In the present invention, a sample to be detected may be selected from the group consisting of a serum sample, a plasma sample, a whole blood sample, a sputum sample, a swab sample, an irrigation solution sample, a fresh tissue sample, a formalin-fixed paraffin-embedded tissue (FFPE) sample, a urine sample, a bacterial culture, a virus culture, a cell line culture, an artificially synthesized plasmid sample and compositions thereof.

In some embodiments, the method of the present invention may be used for the detection of infectious pathogens. For example, it may be used for detecting respiratory tract-related pathogens, including but not limited to detecting one or more selected from the following pathogens: influenza A virus, influenza B virus, respiratory syncytial virus, adenovirus, Mycoplasma pneumonia and novel coronavirus (SARS-CoV-2).

It may be understood that, when the target of a detection object is RNA, the method of the present invention further comprises a step of performing reverse transcription to RNA before the amplifying step. An exemplary reverse transcription reaction conditions may be at 40 °C-60 °C for 1-30 minutes.

### Example 1: Primer sets and flexible probes designed for detection of respiratory tract-related pathogens

In order to verify the effect of the composition of the present invention in detecting influenza A virus (IAV), influenza B virus (IBV), respiratory syncytial virus (RSV), adenovirus (ADV), Mycoplasma pneumonia (MP) and novel coronavirus (SARS-CoV-2), primers and probes in Table 1 are designed below.

**Table 1**

| Name of primer or probe | No. | Nucleoside sequence (5'-3') | Modification |
|---|---|---|---|
| Flexible probe 1 | SEQ ID NO:1 | ACGGATCACAGCGTAAAGC | 5'FAM |
| | | | BHQ1 (14) |
| Flexible probe 2 | SEQ ID NO:2 | ACGGATCACACACGATGACA | 5'ROX |
| | | | BHQ2 (16) |
| Flexible probe 3 | SEQ ID NO:3 | CCOTCTCOCATTAACATATT | 5'Cy5 |
| | | | BHQ2 (17) |
| Upstream primer 1 | SEQ ID NO:4 | ACGGATCACAGCGTAAAGCTGCAACA TGACCAAGGACTGG | / |
| Downstream primer 1 | SEQ ID NO:5 | ATGTAGAAGCCCTGGTAGC | / |
| Upstream primer 2 | SEQ ID NO:6 | ACGGATCACAGCGTAAAGCCTAAGCA GACTCACAGAACT | / |
| Downstream primer 2 | SEQ ID NO:7 | ATGAGAACTTGCCATAGGTT | / |
| Upstream primer 3 | SEQ ID NO:8 | ACGGATCACACACGATGACATAACTC AAGGCGGACGATT | / |
| Downstream primer 3 | SEQ ID NO:9 | TTAACCACGGCGTTCAGG | / |
| Upstream primer 4 | SEQ ID NO: 10 | ACGGATCACACACGATGACATGGACA ACAGCAGACAAC | / |
| Downstream primer 4 | SEQ ID NO:11 | TCCACACGTACAAGGTATCT | / |
| Upstream primer 5 | SEQ ID NO:12 | CCGTCTCGCATTAACATATTATAATCA GGCACGAGAACAG | / |
| Downstream primer 5 | SEQ ID NO:13 | GACTAGCAACCTCCATGGC | / |
| Upstream primer 6 | SEQ ID NO:14 | CCGTCTCGCATTAACATATTAAGCAAC CAACAACATTCATTG | / |
| Downstream primer 6 | SEQ ID NO:15 | GCAATTCAGCATCACAGAC | / |

Among them, the upstream primer 1 and downstream primer 1 are specific primers designed for the target sequence of adenovirus; the upstream primer 2 and downstream primer 2 are specific primers designed for the target sequence of influenza B virus; the upstream primer 3 and the downstream primer 3 are specific primers designed for the target sequence of Mycoplasma pneumoniae; the upstream primer 4 and downstream primer 4 are specific primers designed for the target sequence of novel coronavirus; the upstream primer 5 and the downstream primer 5 are specific primers designed for the target sequence of influenza A virus; and the upstream primer 6 and the downstream primer 6 are specific primers designed for the target sequence of respiratory syncytial virus.

After amplification and extension of the above-described flexible probes, the Tm values of the amplified products are shown in Table 2 below.

**Table 2**

| Fluorescent channel | Melting temperature (Tm, °C) | |
|---|---|---|
| | 76≤Tm<82 | 81≤Tm<86 |
| FAM | IBV | ADV |
| ROX | SARS-CoV-2 | MP |
| Cy5 | RSV | IAV |

### Example 2: Single detections for adenovirus, influenza B virus, respiratory syncytial virus, influenza A virus, Mycoplasma pneumoniae and novel coronavirus

Sample preparation: a vitro transcriptional RNA of each target sequence was used as a positive sample, and each target sequence was subjected to separate detection to verify a detection ability for single virus infection. Pure water was used as a template-free control (NTC).

### Reaction preparation:

After sample preparation, each reaction system was configured according to the ratios described in Table 3 below.

**Table 3**

| Reagent component | Concentration |
|---|---|
| 2× PCR Reaction Buffer (including 3 mM MgCl₂, 30mM Tris-HCl with pH of 8.3 , 0.5mM dNTP, 70mM (NH₄)₂SO₄) | 1× |
| DNA Polymerase | 2U |
| Reverse transcriptase | 5U |
| Upstream primer 1 (SEQ ID NO:4) | 40nM |
| Downstream primer 2 (SEQ ID NO:5) | 240nM |
| Flexible probe 1 (SEQ ID NO:1) | 240nM |
| Upstream primer 2 (SEQ ID NO:6) | 40nM |
| Downstream primer 2 (SEQ ID NO:7) | 240nM |
| Upstream primer 3 (SEQ ID NO:8) | 40nM |
| Downstream primer 3 (SEQ ID NO:9) | 240nM |
| Hybridization probe 2 (SEQ ID NO:2) | 240nM |
| Upstream primer 4 (SEQ ID NO:10) | 40nM |
| Downstream primer 4 (SEQ ID NO:11) | 240nM |
| Upstream primer 5 (SEQ ID NO:12) | 40nM |
| Downstream primer 5 (SEQ ID NO:13) | 240nM |
| Hybridization probe 3 (SEQ ID NO:3) | 240nM |
| Upstream primer 6 (SEQ ID NO:14) | 40nM |
| Downstream primer 6 (SEQ ID NO:15) | 240nM |
| Nucleic acid template | 0.1ng~60ng |
| Ultra-pure water | in a residual quantity until a total volume is 20 µL |

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of a fluorescence quantitative PCR instrument (Suzhou Yarui, MA-6000). The procedure used was as follows: performing reverse transcription at 50 °C for 10 minutes; performing pre-denaturation at 95 °C for 3 minutes; performing, denaturation at 94 °C for 10 seconds, and annealing and extension at 61 °C for 15 seconds, for a total of 10 cycles, without lighting; performing, denaturation at 94 °C for 10 seconds, and annealing and extension at 55 °C for 15 seconds, for a total of 40 cycles, with lighting; and performing, denaturation at 94 °C for 10 seconds, annealing at 50 °C for 90 seconds, and melting curve analysis at 65-95 °C with a temperature rise rate of 0.05 °C/s.

When there is no target to be detected, the single-stranded flexible probe has a curly shape due to molecular flexibility, at this time, the distance between the fluorescence group and the quencher group is relatively close, and the efficiency of fluorescence resonance energy transfer is relatively high. When a target to be detected is present, the upstream and downstream primers will specifically bind with the target sequence and are extended to produce a pre-amplified template, such that the flexible probe may be paired with the pre-amplified template and extended, and the fluorescence group and quencher group will be incorporated into a double-stranded PCR amplified product; and since the change from single strand to double strand will affect the molecular flexibility, at this time, the distance between the fluorescence group and the quencher group will become long, and the efficiency of fluorescence resonance energy transfer will be decreased, such that a fluorescence signal change will be generated and thus may be detected by an instrument. After the completion of PCR amplification, when the melting curve analysis is performed, the double-stranded PCR amplified product will be molten at a certain temperature, such that the product strand with the fluorescence group and the quencher group become single-stranded state, at this time, the molecular flexibility will be restored, the distance between the fluorescence group and the quencher group will become short, and the efficiency of fluorescence resonance energy transfer will be increased, such that a fluorescence signal change will be generated again, which may be used for melting curve analysis.

### Data analysis:

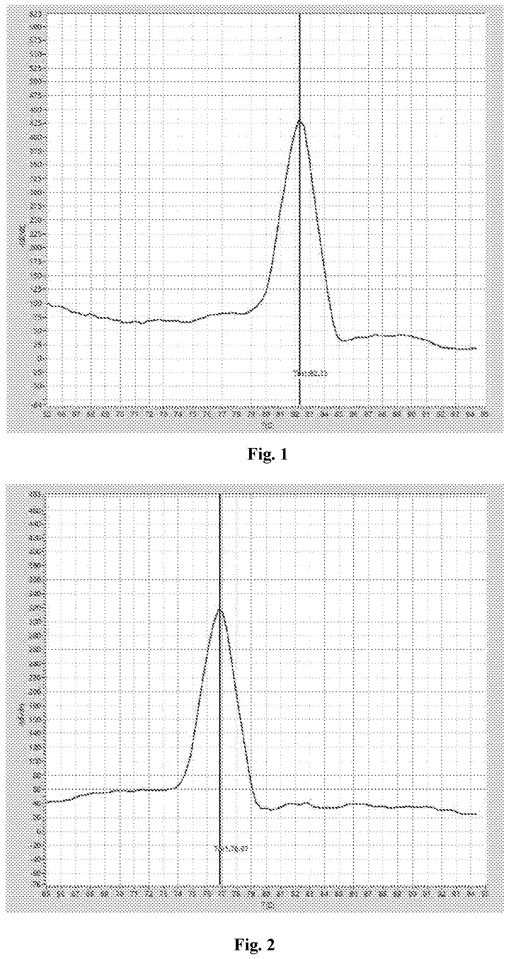

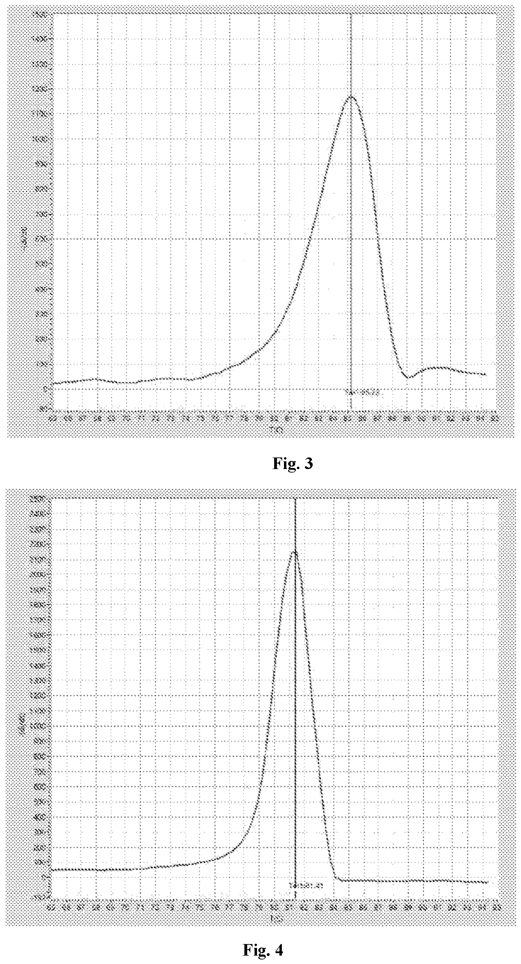

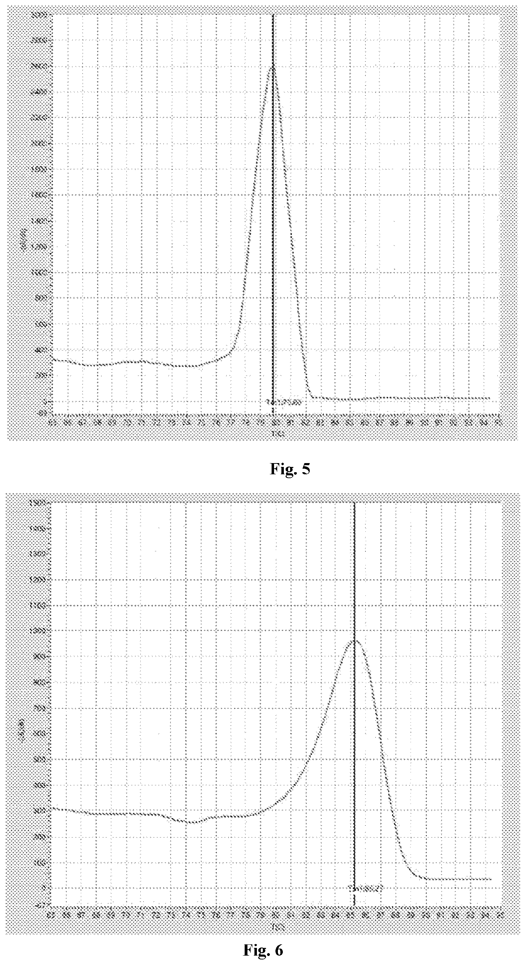

The results are interpreted through the Tm values of the melting curves. Figures 1-6 show views of the melting curves of positive samples of individual respiratory pathogens in multiplex target detection.

It can be seen from Figure-Figure that, for a PCR reaction hole with addition of ADV positive template, a melting peak with Tm value of 82.33 °C is formed in a FAM channel; for a PCR reaction hole with addition of IBV positive template, a melting peak with Tm value of 76.87 °C is formed in a FAM channel; for a PCR reaction hole with addition of MP positive template, a melting peak with Tm value of 85.23- °C is formed in a ROX channel; for a PCR reaction hole with addition of SARS-Cov-2 positive template, a melting peak with Tm value of 81.41 °C is formed in a ROX channel; for a PCR reaction hole with addition of RSV positive template, a melting peak with Tm value of 79.8 °C is formed in a Cy5 channel; and for a PCR reaction hole with addition of IAV positive template, a melting peak with Tm value of 85.27 °C is formed in a Cy5 channel.

### Example 3: Multiplex detections for adenovirus, influenza B virus, respiratory syncytial virus, influenza A virus, Mycoplasma pneumoniae and novel coronavirus

Sample preparation: a vitro transcriptional RNA of each target sequence was used as a positive sample, and two target sequence templates for the same fluorescent channel in Table 2 were mixed for detection, to verify a detection ability of mixed virus infection. Pure water was used as a template-free control (NTC).

### Reaction preparation:

After sample preparation, each reaction system was configured according to the ratios described in Table 3.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of a fluorescence quantitative PCR instrument (Suzhou Yarui, MA-6000). The procedure used was as follows: performing reverse transcription at 50 °C for 10 minutes; performing pre-denaturation at 95 °C for 3 minutes; performing, denaturation at 94 °C for 10 seconds, and annealing and extension at 61 °C for 15 seconds, for a total of 10 cycles, without lighting; performing, denaturation at 94 °C for 10 seconds, and annealing and extension at 55 °C for 15 seconds, for a total of 40 cycles, with lighting; and performing, denaturation at 94 °C for 10 seconds, annealing at 50 °C for 90 seconds, and melting curve analysis at 65-95 °C, with a temperature rise rate of 0.05 °C/s.

### Data analysis:

The results are interpreted through the Tm values of the melting curves.

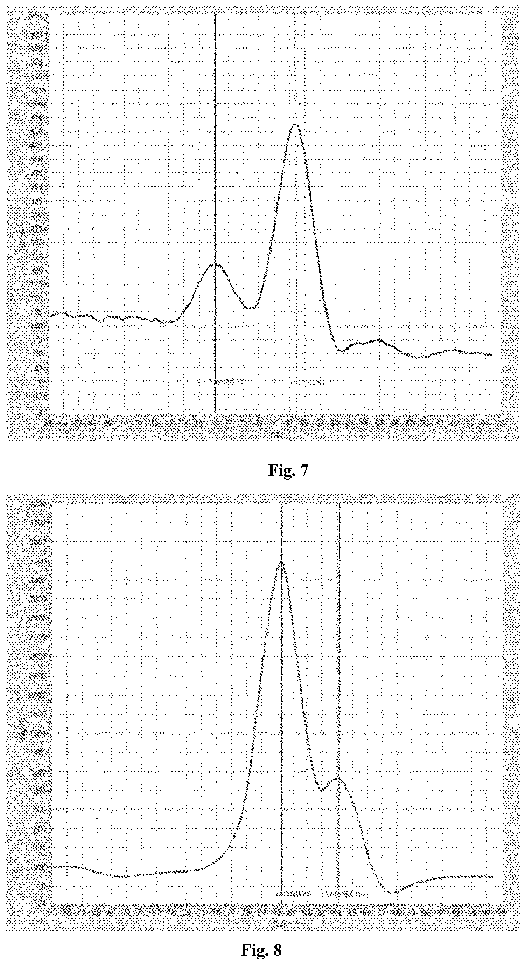

Figures 7-9 show the melting curves of positive samples of individual respiratory viruses in multiplex target detection. It can be seen from Figures 7-9 that, for a PCR reaction hole with co-addition of ADV and IBV positive templates, double melting peaks of ADV and IBV are simultaneously formed in a FAM channel; for a PCR reaction hole with co-addition of MP and SARS-Cov-2 positive templates, double melting peaks of MP and SARS-Cov-2 are simultaneously formed in a ROX channel; and for a PCR reaction hole with co-addition of RSV and IAV positive templates, double melting peaks of RSV and IAV are simultaneously formed in a Cy5 channel. Accordingly, the composition of the present invention has a good specificity in detecting these six respiratory tract-related pathogens, and the obtained melting curves each have a simple pattern, and the Tm values of the two positive targets in the same channel may be clearly distinguished.

### Example 4: Primer sets and hybridization probes designed for detection of respiratory tract-related pathogens

In order to verify the effect of the composition of the present invention in detecting influenza A virus, influenza B virus, respiratory syncytial virus, adenovirus, Mycoplasma pneumonia and novel coronavirus, primers and probes in Table 4 are designed below.

**Table 4**

| Name of primer or probe | No. | Nucleoside sequence (5'-3') | Modification |
|---|---|---|---|
| Upstream primer 1' | SEQ ID NO:16 | CTCCACCACTTACTGATTATCTCAAAGC CGAGATCGCACAG | |
| Downstream primer 1' | SEQ ID NO:17 | TCCCCTTAGTCAGAGGTGACAGG | |
| Upstream primer 2' | SEQ ID NO:18 | CTCCACGACTTACAGATTACTTGAAGG GTTTGAGCCATACTG | |
| Downstream primer 2' | SEQ ID NO:19 | CCCTGGGGTTGAAGGGTAATC | |
| Upstream primer 3' | SEQ ID NO:20 | ACGACCTATGAACGCTCTCTCATTAAG CAAGAAAAGGAAAAGAAG | |
| Downstream primer 3' | SEQ ID NO:21 | TGCAGATCCAACACCTAACAAA | |
| Upstream primer 4' | SEQ ID NO:22 | ACGACCTATGCACGCACTCTCCAACCT ATGAGCAGGCAGGTGGTT | |
| Downstream primer 4' | SEQ ID NO:23 | TGGTAAGGTGACGGCTTTGTAGT | |
| Upstream primer 5' | SEQ ID NO:24 | CAACTAACCAGGATCAACGAGCACCCC AACAGTGAAACGA | |
| Downstream primer 5' | SEQ ID NO:25 | GGCACGAGTAAAACGGCAAA | |
| Upstream primer 6' | SEQ ID NO:26 | CAACCAACCAGCATCAACATGACCGAA AGGTAAGATGGAGA | |
| Downstream primer 6' | SEQ ID NO:27 | ACAGGCAAACTGAGTTGGACG | |
| Hybridization probe 1 | SEQ ID NO:28 | CTCCACCACTTACTGATT | 5'FAM, 3'BHQ1 |
| Hybridization probe 2 | SEQ ID NO:29 | ACGACCTATGAACGCTCTC | 5'ROX |
| | | | 3'BHQ2 |
| Hybridization probe 3 | SEQ ID NO:30 | CAACCAACCAGCATCAAC | 5'Cy5 |
| | | | 3'BHQ2 |

Among them, the upstream primer 1' and the downstream primer 1' are specific primers designed for the target sequence of influenza A virus; the upstream primer 2' and downstream primer 2' are specific primers designed for the target sequence of influenza B virus; the upstream primer 3' and downstream primer 3' are specific primers designed for the target sequence of respiratory syncytial virus; the upstream primer 4' and the downstream primer 4' are specific primers designed for the target sequence of adenovirus; the upstream primer 5' and the downstream primer 5' are specific primers designed for the target sequence of Mycoplasma pneumoniae; and the upstream primer 6' and the downstream primer 6' are specific primers designed for the target sequence of novel coronavirus.

The Tm values of the hybridization probes are shown in Table 5 below.

**Table 5**

| Fluorescent channel | Melting temperature (Tm, °C) | | |
|---|---|---|---|
| | 59≤Tm<62 | 62≤Tm<65 | 72≤Tm<75 |
| FAM | IBV | | IAV |
| ROX | | ADV | RSV |
| Cy5 | | MP | SARS-CoV-2 |

### Example 5: Single detections for adenovirus, influenza B virus, respiratory syncytial virus, influenza A virus, Mycoplasma pneumoniae and novel coronavirus

Sample preparation: a vitro transcriptional RNA of each target sequence was used as a positive sample, and each target sequence was subjected to separate detection to verify a detection ability for single virus infection. Pure water was used as a template-free control (NTC).

### Reaction preparation:

After sample preparation, each reaction system was configured according to the ratios described in Table 6 below.

**Table 6**

| | Reagent component | Concentration |
|---|---|---|
| 2× PCR Reaction Buffer ( including 4.5 mM MgCl₂, 30mM Tris-HCl with pH of 8.3 , 0.8mM dNTP, 50 mM KCl) | | 1× |
| | DNA Polymerase | 2 U |
| | Reverse transcriptase | 5 U |
| | Upstream primer 1' (SEQ ID NO:16) | 45nM |
| | Downstream primer 1' (SEQ ID NO:17) | 250nM |
| | Hybridization probe 1 (SEQ ID NO:28) | 250nM |
| | Upstream primer 2' (SEQ ID NO:18) | 45nM |
| | Downstream primer 2' (SEQ ID NO:19) | 250nM |
| | Upstream primer 3' (SEQ ID NO:20) | 45nM |
| | Downstream primer 3' (SEQ ID NO:21) | 250nM |
| | Hybridization probe 2 (SEQ ID NO:29) | 250nM |
| | Upstream primer 4' (SEQ ID NO:22) | 45nM |
| | Downstream primer 4' (SEQ ID NO:23) | 250nM |
| | Upstream primer 5' (SEQ ID NO:24) | 45nM |
| | Downstream primer 5' (SEQ ID NO:25) | 250nM |
| | Hybridization probe 3 (SEQ ID NO:30) | 250nM |
| | Upstream primer 6' (SEQ ID NO:26) | 45nM |
| | Downstream primer 6' (SEQ ID NO:27) | 250nM |
| | Nucleic acid template | 0.1ng~60ng |
| | Ultra-pure water | in a residual quantity until a total volume is 20 µL |

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of a fluorescence quantitative PCR instrument (Suzhou Yarui, MA-6000). The procedure used was as follows: performing reverse transcription at 50 °C for 10 minutes; performing pre-denaturation at 95 °C for 3 minutes; performing, denaturation at 95 °C for 10 seconds, and annealing and extension at 60 °C for 15 seconds, for a total of 10 cycles, without lighting; performing, denaturation at 95 °C for 10 seconds, and annealing and extension at 56 °C for 15 seconds, for a total of 40 cycles, with lighting; and performing, denaturation at 94 °C for 10 seconds, annealing at 50 °C for 120 seconds, and melting curve analysis at 50-90 °C, with a temperature rise rate of 0.03 °C/s.

When there is no target to be detected, the hybridization probe is in single-stranded state, at this time, the distance between the fluorescence group and the quencher group is relatively close, and the efficiency of fluorescence resonance energy transfer is relatively high. When a target to be detected is present, the upstream and downstream primers will specifically bind with the target sequence and are extended to produce an amplified template, such that the hybridization probe may be hybridized with the amplified template, resulting in a change of the distance between the fluorescence group and the quencher group and a change of the efficiency of fluorescence resonance energy transfer, and thus a fluorescence signal change will be generated and thus may be detected by an instrument. After the completion of PCR amplification, when the melting curve analysis is performed, the hybridization probe hybridized with the amplified product will be molten at a certain temperature and separated from the product strand, such that the distance between the fluorescence group and the quencher group changes, at this time, the efficiency of fluorescence resonance energy transfer changes, further producing a fluorescence signal change, which may be detected by the instrument and may be used for the melting curve analysis.

### Data analysis:

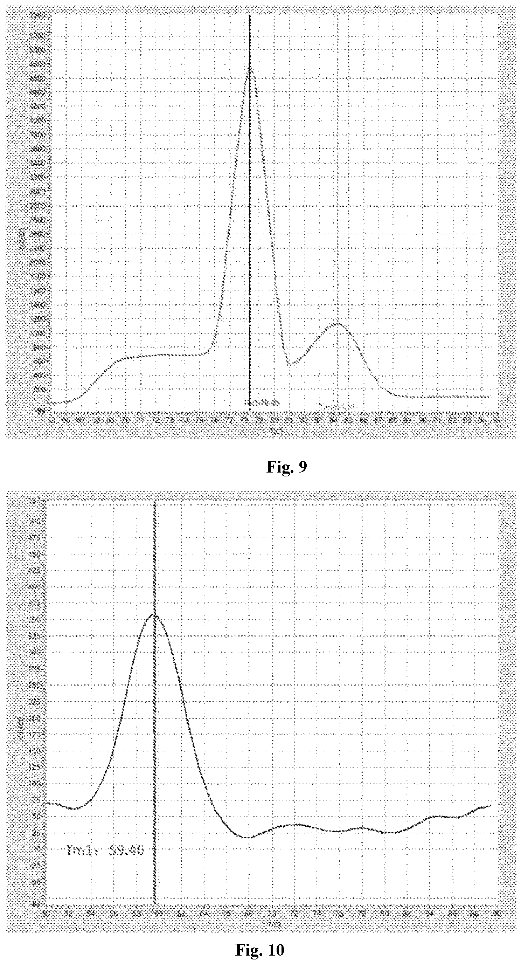

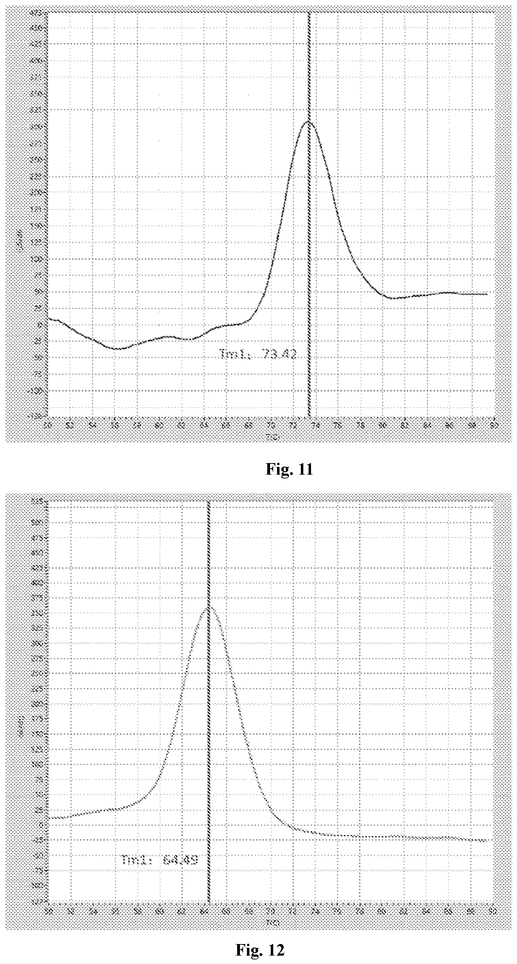

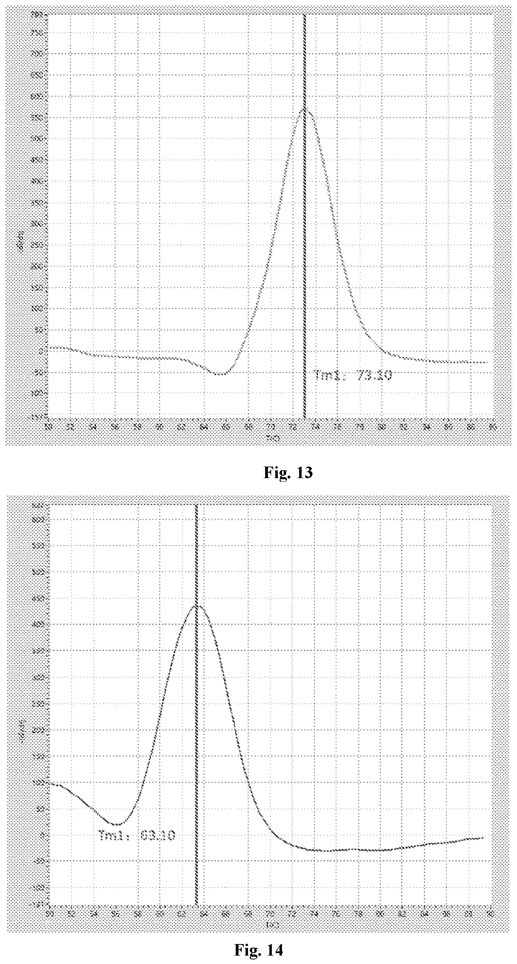

The results are interpreted through the Tm values of the melting curves. Figures 10-15 show the melting curves of positive samples of individual respiratory viruses in multiplex target detection.

It can be seen from Figures 10-15 that, for a PCR reaction hole with addition of IBV positive template, a melting peak with Tm value of 59.46 °C is formed in a FAM channel; for a PCR reaction hole with addition of IAV positive template, a melting peak with Tm value of 73.42 °C is formed in a FAM channel; for a PCR reaction hole with addition of ADV positive template, a melting peak with Tm value of 64.49 °C is formed in a ROX channel; for a PCR reaction hole with addition of RSV positive template, a melting peak with Tm value of 73.10 °C is formed in a ROX channel; for a PCR reaction hole with addition of MP positive template, a melting peak with Tm value of 63.10 °C is formed in a Cy5 channel; and for a PCR reaction hole with addition of SARS-CoV-2 positive template, a melting peak with Tm value of 74.48 °C is formed in a Cy5 channel.

### Example 6: Multiplex detections for adenovirus, influenza B virus, respiratory syncytial virus, influenza A virus, Mycoplasma pneumoniae and novel coronavirus

Sample preparation: a vitro transcriptional RNA of each target sequence was used as a positive sample, and two target sequence templates for the same fluorescent channel in Table 5 were mixed for detection, to verify a detection ability of mixed virus infection. TE Buffer was used as a template-free control (NTC).

### Reaction preparation:

After sample preparation, each reaction system was configured according to the ratios described in Table 6.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of a fluorescence quantitative PCR instrument (Suzhou Yarui, MA-6000). The procedure used was as follows: performing reverse transcription at 50 °C for 10 minutes; performing pre-denaturation at 95 °C for 3 minutes; performing, denaturation at 95 °C for 10 seconds, and annealing and extension at 60 °C for 15 seconds, for a total of 10 cycles, without lighting; performing, denaturation at 95 °C for 10 seconds, and annealing and extension at 56 °C for 15 seconds, for a total of 40 cycles, with lighting; and performing, denaturation at 94 °C for 10 seconds, annealing at 50 °C for 120 seconds, and melting curve analysis at 50-90 °C, with a temperature rise rate of 0.03 °C/s.

### Data analysis:

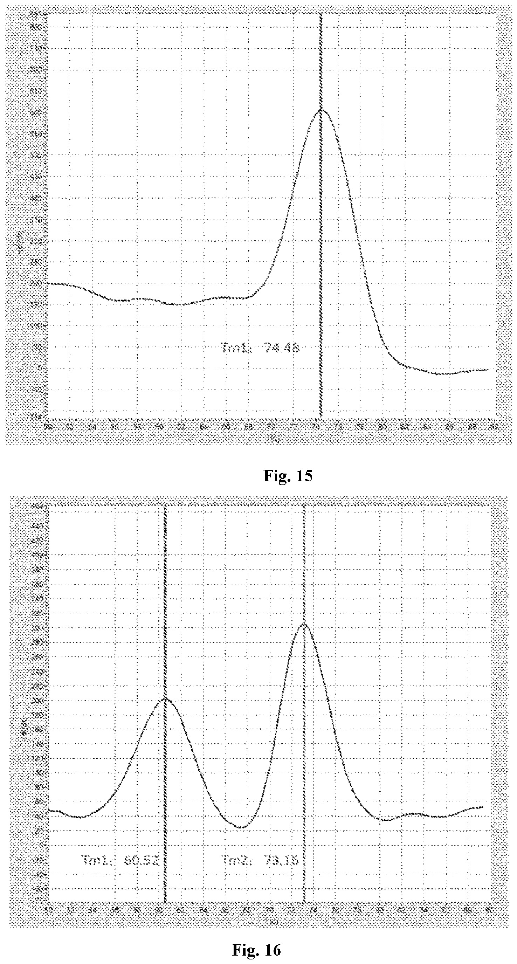

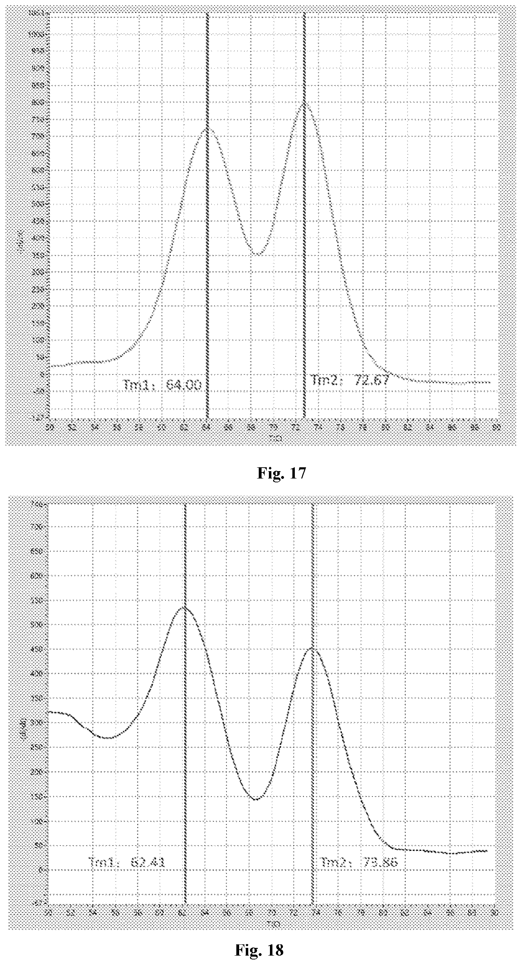

The results are interpreted through the Tm values of the melting curves. Figures 16-18 show the melting curves of positive samples of individual respiratory viruses in multiplex target detection.

It can be seen from Figures 16-18 that, for a PCR reaction hole with co-addition of IBV and IAV positive templates, double melting peaks of IBV and IAV are simultaneously formed in a FAM channel; for a PCR reaction hole with co-addition of ADV and RSV positive templates, double melting peaks of ADV and RSV are simultaneously formed in a ROX channel; and for a PCR reaction hole with co-addition of MP and SARS-CoV-2 positive templates, double melting peaks of MP and SARS-CoV-2 are simultaneously formed in a Cy5 channel. Accordingly, the composition of the present invention has a good specificity in detecting these six respiratory tract-related pathogens, and the obtained melting curves each have a simple pattern, and the Tm values of the two positive targets in the same channel may be clearly distinguished.

### Example 7: Single detections for adenovirus, influenza B virus, respiratory syncytial virus, influenza A virus, Mycoplasma pneumoniae and novel coronavirus

Sample preparation: a vitro transcriptional RNA of each target sequence was used as a positive sample, and each target sequence was subjected to separate detection to verify a detection ability for single virus infection. TE Buffer was used as a template-free control (NTC).

### Reaction preparation:

After sample preparation, each reaction system was configured according to the ratios described in Table 6 below.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of a fluorescence quantitative PCR instrument (Shanghai Hongshi, SLAN^{®}-96P). The procedure used was as follows: performing reverse transcription at 50 °C for 10 minutes; performing pre-denaturation at 95 °C for 3 minutes; performing, denaturation at 95 °C for 10 seconds, and annealing and extension at 60 °C for 15 seconds, for a total of 10 cycles, without lighting; performing, denaturation at 95 °C for 10 seconds, and annealing and extension at 56 °C for 15 seconds, for a total of 40 cycles, with lighting; and performing, denaturation at 94 °C for 10 seconds, annealing at 50 °C for 120 seconds, and melting curve analysis at 50-90 °C, with a temperature rise rate of 0.03 °C/s.

When there is no target to be detected, the hybridization probe is in single-stranded state, at this time, the distance between the fluorescence group and the quencher group is relatively close, and the efficiency of fluorescence resonance energy transfer is relatively high. When a target to be detected is present, the upstream and downstream primers will specifically bind with the target sequence and are extended to produce an amplified template, such that the hybridization probe can be hybridized with the amplified template, resulting in a change of the distance between the fluorescence group and the quencher group and a change of the efficiency of fluorescence resonance energy transfer, and thus a fluorescence signal change will be generated and thus may be detected by an instrument. After the completion of PCR amplification, when the melting curve analysis is performed, the hybridization probe hybridized with the amplified product will be molten at a certain temperature and separated from the product strand, such that the distance between the fluorescence group and the quencher group changes, at this time, the efficiency of fluorescence resonance energy transfer changes, further producing a fluorescence signal change, which may be detected by the instrument and may be used for the melting curve analysis.

### Data analysis:

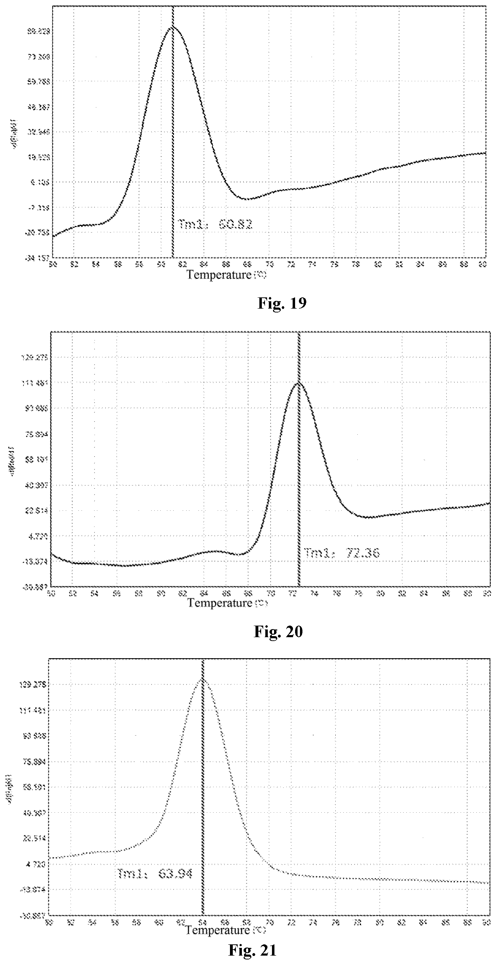

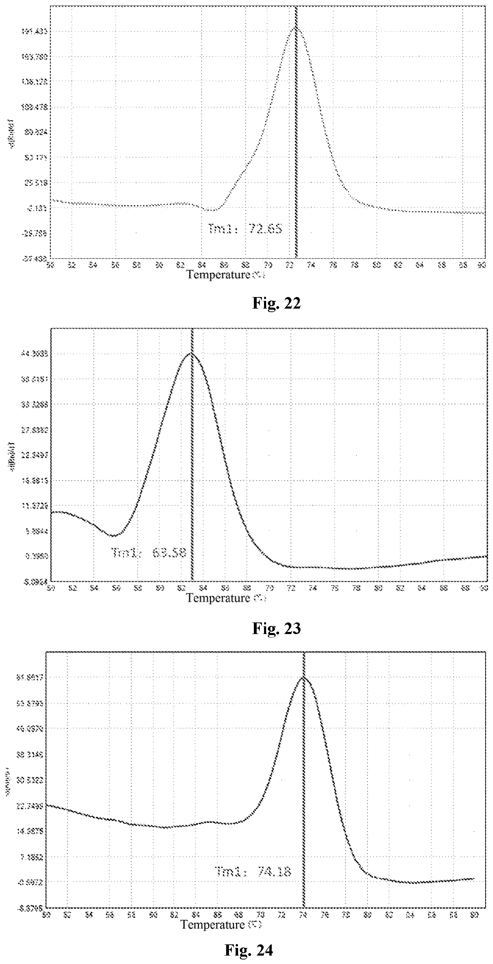

The results are interpreted through the Tm values of the melting curves. Figures 19-24 show the melting curves of positive samples of individual respiratory viruses in multiplex target detection.

It can be seen from Figures 19-24 that, for a PCR reaction hole with addition of IBV positive template, a melting peak with Tm value of 60.82 °C is formed in a FAM channel; for a PCR reaction hole with addition of IAV positive template, a melting peak with Tm value of 72.36 °C is formed in a FAM channel; for a PCR reaction hole with addition of ADV positive template, a melting peak with Tm value of 63.94 °C is formed in a ROX channel; for a PCR reaction hole with addition of RSV positive template, a melting peak with Tm value of 72.65 °C is formed in a ROX channel; for a PCR reaction hole with addition of MP positive template, a melting peak with Tm value of 63.58 °C is formed in a Cy5 channel; and for a PCR reaction hole with addition of SARS-CoV-2 positive template, a melting peak with Tm value of 74.18 °C is formed in a Cy5 channel.

### Example 8: Multiplex detections for adenovirus, influenza B virus, respiratory syncytial virus, influenza A virus, Mycoplasma pneumoniae and novel coronavirus

Sample preparation: a vitro transcriptional RNA of each target sequence was used as a positive sample, and two target sequence templates for the same fluorescent channel in Table 5 were mixed for detection, to verify a detection ability of mixed virus infection. TE Buffer was used as a template-free control (NTC).

### Reaction preparation:

After sample preparation, each reaction system was configured according to the ratios described in Table 6.

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of a fluorescence quantitative PCR instrument (Shanghai Hongshi, SLAN^{®}-96P). The procedure used was as follows: performing reverse transcription at 50 °C for 10 minutes; performing pre-denaturation at 95 °C for 3 minutes; performing, denaturation at 95 °C for 10 seconds, and annealing and extension at 60 °C for 15 seconds, for a total of 10 cycles, without lighting; performing, denaturation at 95 °C for 10 seconds, and annealing and extension at 56 °C for 15 seconds, for a total of 40 cycles, with lighting; and performing, denaturation at 94 °C for 10 seconds, annealing at 50 °C for 120 seconds, and melting curve analysis at 50-90 °C, with a temperature rise rate of 0.03 °C/s.

### Data analysis:

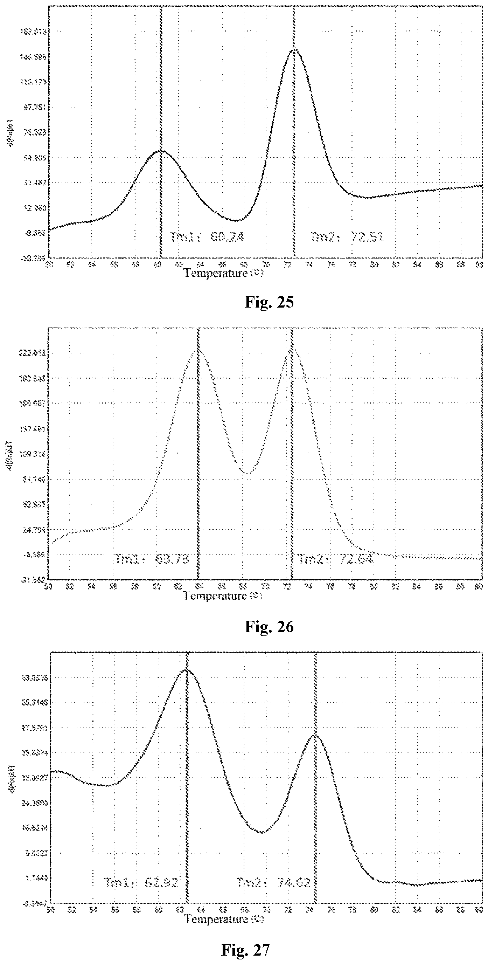

The results are interpreted through the Tm values of the melting curves. Figures 25-27 show the melting curves of positive samples of individual respiratory viruses in multiplex target detection.

It can be seen from Figures 25-27 that, for a PCR reaction hole with co-addition of IAV and IBV positive templates, double melting peaks of IAV and IBV are simultaneously formed in a FAM channel; for a PCR reaction hole with co-addition of ADV and RSV positive templates, double melting peaks of ADV and RSV are simultaneously formed in a ROX channel; and for a PCR reaction hole with co-addition of MP and SARS-CoV-2 positive templates, double melting peaks of MP and SARS-CoV-2 are simultaneously formed in a Cy5 channel. Accordingly, the composition of the present invention has good specificity in detecting six respiratory tract-related pathogens, and the obtained melting curves each have a simple pattern, and the Tm values of the two positive targets in the same channel may be clearly distinguished.

### Example 9 Multiplex detections for adenovirus, influenza B virus, respiratory syncytial virus, influenza A virus, rhinovirus, Mycoplasma pneumoniae and novel coronavirus

In order to verify the effect of the composition of the present invention in detecting influenza A virus, influenza B virus, respiratory syncytial virus, adenovirus, rhinovirus (Rhv), Mycoplasma pneumonia and novel coronavirus, primers and probe in Table 7 are designed below. Meanwhile, a quality monitoring is added to the detections for respiratory tract-related pathogens to ensure that there are no systematic errors in the process of PCR. An internal quality control is directed at a pair of primers and a probe designed for humanized glyceraldehyde-3-phosphate dehydrogenase (GAPDH) gene. The probe is a hydrolysis probe, and thus no melting curve will be generated.

**Table 7**

| Name of primer or probe | No. | Nucleoside sequence (5'-3') | Modification |
|---|---|---|---|
| Upstream primer 7' | SEQ ID NO:31 | CAACCAACCAGCATGAAC CGCTTGAATATGTTGATATGCTCCA | |
| Downstream primer 7' | SEQ ID NO:32 | GCGGATAACGGTATCTGTTGTTT | |
| Upstream primer 8' | SEQ ID NO:33 | GGAGCGAGATCCCTCCAAAAT | |
| Downstream primer 8' | SEQ ID NO:34 | GGCTGTTGTCATACTTCTCATGG | |
| Hydrolysis probe 1 | SEQ ID NO:35 | GCAGGGGGGAGCCAAAAGGGT | 5'VIC |
| | | | 3'BHQ1 |
| Upstream primer 9' | SEQ ID NO:36 | CAACTAACCAGCATCAACGAGCACCCC AACAGTGAAACGA | |

Among them, the specific primers and probes of influenza A virus, influenza B virus, respiratory syncytial virus, adenovirus, and novel coronavirus are shown in Table 4; and the downstream primer of Mycoplasma pneumoniae is shown in Table 4. The rest primers and probe are shown in Table 7, wherein the upstream primer 7' and the downstream primer 7' are specific primers designed for the target sequence of rhinovirus; the upstream primer 8', the downstream primer 8' and the hydrolysis probe 1 are specific primers and probe designed for humanrized glyceraldehyde-3-phosphate dehydrogenase (GAPDH) gene; and the upstream primer 9' is a specific primer designed for the target sequence of Mycoplasma pneumoniae.

Among them, the Tm values of the hybridization probes are shown in Table 8 below.

**Table 8**

| Fluorescent channel | Melting temperature (Tm, °C) | | |
|---|---|---|---|
| | 60≤Tm<65 | 65≤Tm<70 | 70≤Tm<75 |
| FAM | IBV | | IAV |
| ROX | | ADV | RSV |
| Cy5 | RhV | MP | SARS-CoV-2 |

Sample preparation: a vitro transcriptional RNA of each target sequence was used as a positive sample, and each target sequence was subjected to separate detection to verify a detection ability for single virus infection. Pure water was used as a template-free control (NTC).

### Reaction preparation:

After sample preparation, each reaction system was configured according to the ratios described in Table 9.

**Table 9**

| | Reagent component | Concentration |
|---|---|---|
| 2× PCR Reaction Buffer (including 4.5 mM MgCl₂,30mM Tris-HCl with pH of 8.3 , 0.8mM dNTP, 50 mM KCl) | | 1× |
| | DNA Polymerase | 2 U |
| | Reverse transcriptase | 5 U |
| | Upstream primer 1' (SEQ ID NO:16) | 45nM |
| | Downstream primer 1' (SEQ ID NO:17) | 250nM |
| | Hybridization probe 1 (SEQ ID NO:28) | 250nM |
| | Upstream primer 2' (SEQ ID NO:18) | 45nM |
| | Downstream primer 2' (SEQ ID NO:19) | 250nM |
| | Upstream primer 3' (SEQ ID NO:20) | 45nM |
| | Downstream primer 3' (SEQ ID NO:21) | 250nM |
| | Hybridization probe 2 (SEQ ID NO:29) | 250nM |
| | Upstream primer 4' (SEQ ID NO:22) | 45nM |
| | Downstream primer 4' (SEQ ID NO:23) | 250nM |
| | Upstream primer 9' (SEQ ID NO:36) | 45nM |
| | Downstream primer 5' (SEQ ID NO:25 ) | 250nM |
| | Hybridization probe 3 (SEQ ID NO:30) | 250nM |
| | Upstream primer 6' (SEQ ID NO:26) | 45nM |
| | Downstream primer 6' (SEQ ID NO:27) | 250nM |
| | Upstream primer 7' (SEQ ID NO:31) | 45nM |
| | Downstream primer 7' (SEQ ID NO:32) | 250nM |
| | Upstream primer 8' (SEQ ID NO:33) | 200nM |
| | Downstream primer 8' (SEQ ID NO:34) | 200nM |
| | Hydrolysis probe 1 (SEQ ID NO:35) | 100nM |
| | Nucleic acid template | 0.1ng~60ng |
| | Ultra-pure water | in a residual quantity until a total volume is 20 µL |

### PCR amplification and melting curve analysis:

After a PCR tube was sealed by a cap, the sample was gently and uniformly mixed, and centrifuged for a short time and then left for 5 min at a room temperature. The PCR tube was put in a handheld centrifuge again, centrifuged for a short time, and then transferred to a tray of a fluorescence quantitative PCR instrument (Shanghai Hongshi, SLAN^{®}-96P). The procedure used was as follows: performing reverse transcription at 50 °C for 10 minutes; performing pre-denaturation at 95 °C for 3 minutes; performing, denaturation at 95 °C for 10 seconds, and annealing and extension at 60 °C for 15 seconds, for a total of 10 cycles, without lighting; performing, denaturation at 95 °C for 10 seconds, and annealing and extension at 56 °C for 15 seconds, for a total of 40 cycles, with lighting; and performing, denaturation at 94 °C for 10 seconds, annealing at 50 °C for 120 seconds, and melting curve analysis at 50-90 °C, with a temperature rise rate of 0.03 °C/s.

### Data analysis:

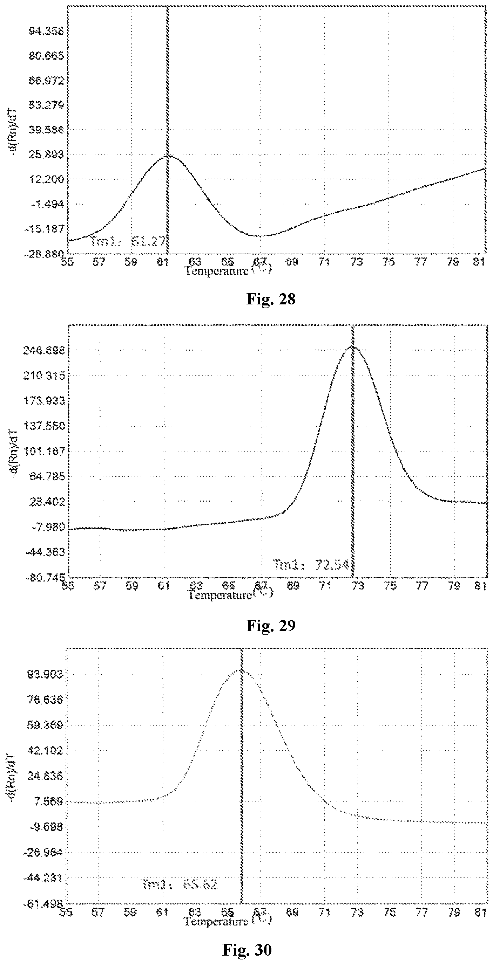

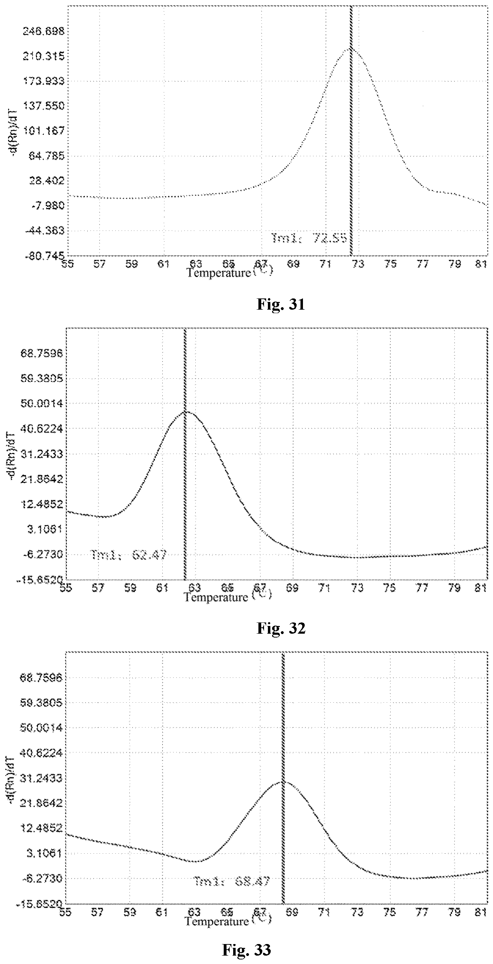

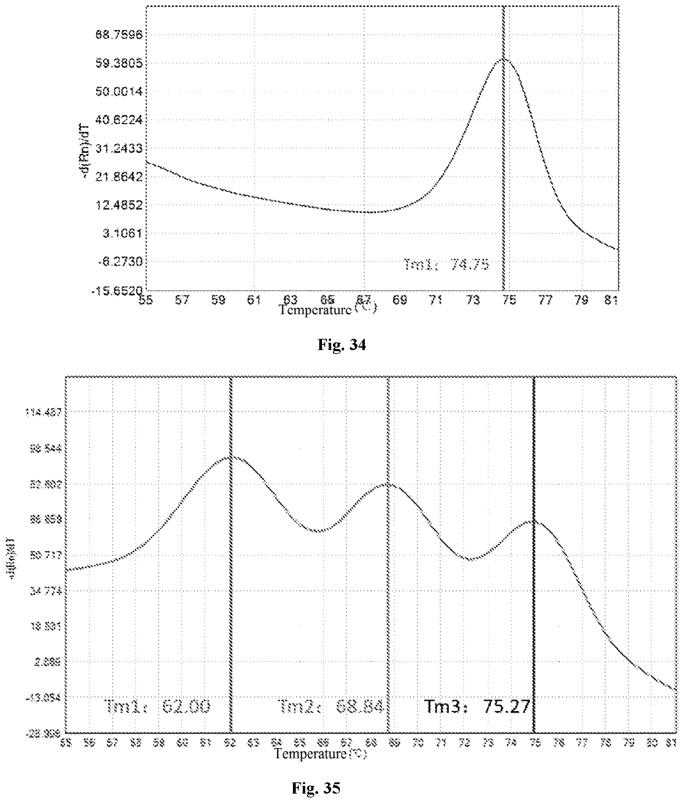

The results are interpreted through the Tm values of the melting curves. Figures 28-35 show the melting curves of positive samples of individual respiratory viruses in multiplex target detection.

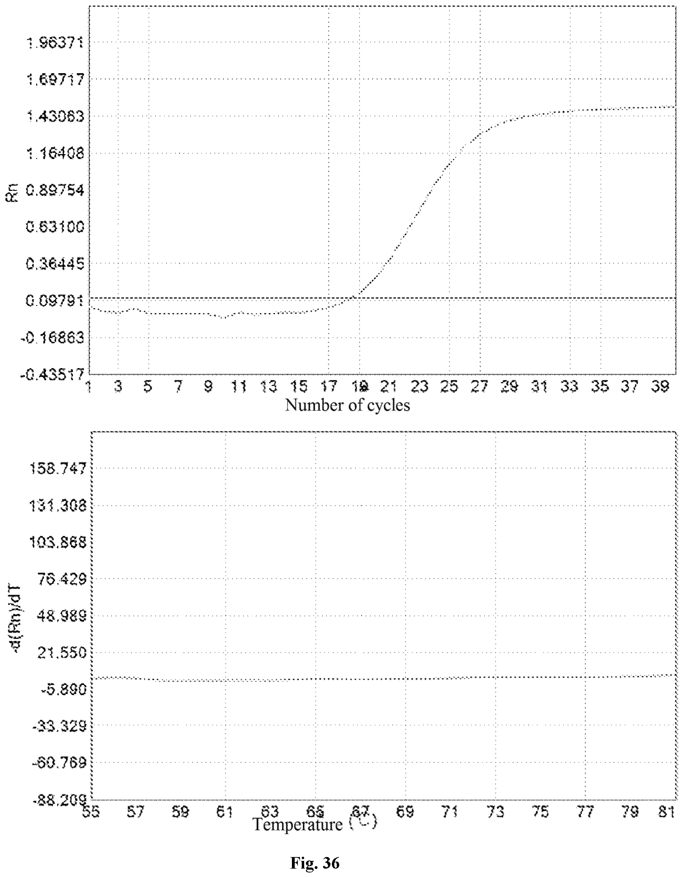

It can be seen from Figures 28-35 that, for a PCR reaction hole with addition of IBV positive template, a melting peak with Tm value of 61.27 °C is formed in a FAM channel; for a PCR reaction hole with addition of IAV positive template, a melting peak with Tm value of 72.54 °C is formed in a FAM channel; for a PCR reaction hole with addition of ADV positive template, a melting peak with Tm value of 65.62 °C is formed in a ROX channel; for a PCR reaction hole with addition of RSV positive template, a melting peak with Tm value of 72.55 °C is formed in a ROX channel; for a PCR reaction hole with addition of RhV positive template, a melting peak with Tm value of 62.47 °C is formed in a Cy5 channel; for a PCR reaction hole with addition of MP positive template, a melting peak with Tm value of 68.47 °C is formed in a Cy5 channel; and for a PCR reaction hole with addition of SARS-CoV-2 positive template, a melting peak with Tm value of 74.75 °C is formed in a Cy5 channel. For a PCR reaction hole with co-addition of RhV, MP and SARS-CoV-2 positive templates, triple melting peaks of RhV, MP and SARS-CoV-2 are simultaneously formed in a Cy5 channel. In addition, it can be seen from Figure 36 that, for a PCR reaction hole with addition of GAPDH positive template, a significant S-type amplification curve (upper side) is formed in a VIC channel, but no melting peak (lower side) is generated.

## Claims

1. A multiplex nucleic acid detection method, comprising the following steps:
mixing a composition containing a first primer set and a first probe with a sample from a subject;
amplifying a target sequence that may be present in the sample;
performing a melting curve analysis to an amplified product in a corresponding detection channel;
and judging whether one or more targets are present, according to a result of the melting curve analysis,
wherein, the first primer set comprises an upstream primer and a downstream primer both of which are specific to a first target, and an upstream primer and a downstream primer both of which are specific to a second target,
wherein, the upstream primer specific to the first target or the downstream primer specific to the first target comprises a first signal detection region located upstream of a target sequence binding region,
wherein, the upstream primer specific to the second target or the downstream primer specific to the second target comprises a second signal detection region located upstream of a target sequence binding region,
wherein, the first signal detection region and the second signal detection region are designed to be not complementarily paired with a target sequence,
and wherein, all or part of a sequence of the first probe is the same as those of the first signal detection region and the second signal detection region, so as to be capable of forming double-stranded structures with complementary sequences of the first signal detection region and the second signal detection region respectively; and the first probe comprises a first detection group and a second detection group, and the first detection group and the second detection group can generate signal change after being incorporated into the double-stranded structures.

2. The method according to claim 1, wherein, the composition further comprises a second primer set and a second probe,
the second primer set comprises an upstream primer and a downstream primer both of which are specific to a third target, and an upstream primer and a downstream primer both of which are specific to a fourth target,
wherein, the upstream primer specific to the third target or the downstream primer specific to the third target comprises a third signal detection region located upstream of a target sequence binding region; and the upstream primer specific to the fourth target or the downstream primer specific to the fourth target comprises a fourth signal detection region located upstream of a target sequence binding region,
wherein, the third signal detection region and the fourth signal detection region are designed to be not complementarily paired with a target sequence,
and wherein, all or part of the sequence of the second probe is the same as those of the third signal detection region and the fourth signal detection region, so as to be capable of forming double-stranded structures with complementary sequences of the third signal detection region and the fourth signal detection region respectively; the second probe comprises a third detection group and a fourth detection group, and the third detection group and the fourth detection group can generate signal change after being incorporated into the double-stranded structures; and a signal generated by the second probe corresponds to a different detection channel from that of a signal generated by the first probe.

3. The method according to claim 1, wherein, the first probe and the second probe are selected from the group consisting of the following: an oligonucleotide that can form a curly shape due to molecular flexibility in single-stranded state, an oligonucleotide that can form a hairpin structure in single-stranded state, an oligonucleotide that can form a stem-loop structure in single-stranded state, an oligonucleotide that can form a pseudoknot structure in single-stranded state, and an oligonucleotide that can form a triplex structure in single-stranded state.

4. The method according to any one of claims 1-3, wherein, the first detection group and the second detection group are spaced apart from each other by 5-25 bases and are not at a 3' end; and the third detection group and the fourth detection group are spaced apart from each other by 5-25 bases and are not at a 3' end.

5. The method according to claim 4, wherein, the first probe can amplify amplified products of the first primer set, but cannot amplify amplified products of other primer sets; and the second probe can amplify amplified products of the second primer set, but cannot amplify amplified products of other primer sets.

6. The method according to claim 4, wherein, the signal detection region is spaced apart from the target sequence binding region on the primer, on which the signal detection region is located, by 0-40 bases.

7. The method according to claim 4, wherein, the first signal detection region is the same as the second signal detection region.

8. The method according to claim 6, wherein, the spacing is designed to allow different amplified products of the first probe to have different melting temperatures; and the spacing is designed to allow different amplified products of the second probe to have different melting temperatures.

9. The method according to any one of claims 1-3, wherein, the first probe can be hybridized with amplified products of the first primer set, but cannot be hybridized with amplified products of other primer sets; the second probe can be hybridized with amplified products of the second primer set, but cannot be hybridized with amplified products of other primer sets; and 3' ends of the first probe and the second probe are designed to be not extended as primers.

10. The method according to claim 9, wherein, the first detection group or the second detection group on the first probe or the second probe is located at a 3' end thereof.

11. The method according to claim 9, wherein the 3' ends of the first probe and the second probe are blocked.

12. The method according to claim 10 or 11, wherein, the first detection group is spaced apart from the second detection group by 5-25 bases; and the third detection group is spaced apart from the fourth detection group by 5-25 bases.

13. The method according to claim 9, wherein, the signal detection region is spaced apart from the target sequence binding region on the primer, on which the signal detection region is located, by 0-10 bases.

14. The method according to claim 9, wherein, the first signal detection region and the second signal detection region are designed to allow hybridization products of their complementary sequences with the first probe to have different melting temperatures; and the third signal detection region and the fourth signal detection region are designed to allow hybridization products of their complementary sequences with the second probe to have different melting temperatures.

15. A composition for multiplex nucleic acid detection, comprising:
a first primer set, comprising an upstream primer and a downstream primer both of which are specific to a first target, and an upstream primer and a downstream primer both of which are specific to a second target; and
a first probe, all or part of the sequence thereof is the same as those of a first signal detection region and a second signal detection region, and can form double-stranded structures with complementary sequences of the first signal detection region and the second signal detection region respectively; the first probe comprises a first detection group and a second detection group, and the first detection group and the second detection group can generate signal change after being incorporated into the double-stranded structures,
wherein, the first signal detection region is located upstream of a target sequence binding region of the upstream primer specific to the first target or the downstream primer specific to the first target, and the second signal detection region is located upstream of a target sequence binding region of the upstream primer specific to the second target or the downstream primer specific to the second target; and the first signal detection region and the second signal detection region are designed to be not complementarily paired with a target sequence.

16. The composition according to claim 15, wherein, the composition further comprises a second primer set and a second probe,
the second primer set comprises an upstream primer and a downstream primer both of which are specific to a third target, and an upstream primer and a downstream primer both of which are specific to a fourth target,
wherein, the upstream primer specific to the third target or the downstream primer specific to the third target comprises a third signal detection region located upstream of a target sequence binding region; and the upstream primer specific to the fourth target or the downstream primer specific to the fourth target comprises a fourth signal detection region located upstream of a target sequence binding region,
wherein, the third signal detection region and the fourth signal detection region are designed to be not complementarily paired with a target sequence,
and wherein, all or part of the sequence of the second probe is the same as those of the third signal detection region and the fourth signal detection region, so as to be capable of forming double-stranded structures with complementary sequences of the third signal detection region and the fourth signal detection region respectively; the second probe comprises a third detection group and a fourth detection group, and the third detection group and the fourth detection group can generate signal change after being incorporated into the double-stranded structures; and a type of a signal generated by the second probe is different from that of a signal generated by the first probe.

17. The composition according to claim 15, wherein, the first probe and the second probe are selected from the group consisting of the following: an oligonucleotide that can form a curly shape due to molecular flexibility in single-stranded state, an oligonucleotide that can form a hairpin structure in single-stranded state, an oligonucleotide that can form a stem-loop structure in single-stranded state, an oligonucleotide that can form a pseudoknot structure in single-stranded state, and an oligonucleotide that can form a triplex structure in single-stranded state.

18. The composition according to any one of claims 15-17, wherein, the first probe can amplify amplified products of the first primer set, but cannot amplify amplified products of other primer sets; and the second probe can amplify amplified products of the second primer set, but cannot amplify amplified products of other primer sets.

19. The composition according to any one of claims 15-17, wherein, the first probe can be hybridized with amplified products of the first primer set, but cannot be hybridized with amplified products of other primer sets; the second probe can be hybridized with amplified products of the second primer set, but cannot be hybridized with amplified products of other primer sets; and 3' ends of the first probe and the second probe are designed to be not extended as primers.

20. A kit, comprising the primer set and the probe as defined in any one of claims 1-9.
